# EUROPEAN PATENT APPLICATION

(11) **EP 0 962 773 A1**
(43) Date of publication of application: **08.12.1999**
(21) Application number: 98430015.2
(22) Date of filing: 03.06.1998
(51) Int. Cl.: G01N 33/553

(54) **Electrochemical based assay processes instrument and labels**

(71) Applicant: Jones, Mark Howard, 34130 Saint-Aunes (FR)
(72) Inventor: Jones, Mark Howard, 34130 Saint-Aunes (FR)
(74) Representative: Domange, Maxime

(57) **Abstract**

A method for quantitating or detecting an analyte using an electrochemical reaction, comprising a material in contact with an electrolyte in a cell or chamber but not in electronic contact with an external electronic circuit, at least some of said material having at least its surface conducting or semi-conducting, and applying a voltage gradient across at least a portion of said material, the size of said material, the conductivity of said electrolyte and/or reactant liquid and the voltage gradient being such that not only are anodic and cathodic faces established in respect of at least some of the conducting or semi-conducting material, rendering said material bipolar, the electropotentials so generated are such that said electrochemical reaction takes place on at least a portion of the surfaces of said material resulting in the production of a detectable signal or detectable material. The said bipolar material which, advantageously, are spherical or fibres, may comprise solid conducting or semi-conducting material or cores of non-conducting material with coatings of conducting or non-conducting material thereon. The material, which are preferably in suspension, may also be bound or captured directly or indirectly onto a surface or filter matrix. The said surface or filter matrix if conducting is not acting as an electrode to carry out electrochemistry. The detection of the electrochemically-generated signal or material is related to the quantity or presence of said analyte.

## Description

### Field of the Invention

This invention relates to an instrument, a method and labels for carrying out assays using an electrochemical reaction on a conducting or semi-conducting material in contact with an electrolyte but not in electronic contact with an external electronic circuit.

### Background of the Invention

The need for methods that provide rapid, sensitive, specific, precise and low-cost detection of numerous analytes has led to the development of a large number of differing assay methods and technologies. The desire and need to improve these current methods still exists and numerous companies are working towards the development of improved means and the exploration of new methods. The potential use of electrochemistry for the development of assays which are rapid, sensitive, specific, precise and low-cost has not escaped study due in part to this technique's ability to detect specific electronic properties directly caused by reactions at the surface of an electrode electronically connected to an external electronic circuit.

The use of particles, beads or other structures suspended in assay buffers is now almost universal in immunoassay analyser systems available; examples are ELECSYS (Boehringer Mannheim), copalis technology (Sienna Biotech, Columbia MD), Luminex (Austin TX) with the fluorescence bead multiplex analysis system, IMMULITE (Diag Prod.Corp), ACS:180 (Ciba Corning), Cobas Core (Roche), ACCESS (Sanofi), AIA-1200 DX (Tosoh), ACAplus (DuPont) and Immuno 1 (Bayer). The reasons for using particles, beads or other structures suspended in assay buffers is that they allow for rapid kinetics and also facilitate the manufacturing and automation of random access assay systems. These also present some disadvantages related to keeping the beads in suspension, washing is not easy and problems can arise from the particles, beads or other structures suspended in assay buffers interfering with the detection systems.

When considering the detection and assay of analytes in gels, various methods are used to achieve a photographic-like analysis of the spatial distribution of samples within the gel. Typically, this is achieved by staining the gel directly with various agents to visualise the molecules in the gel, for example commassie blue is used to detect proteins in gels. The proteins bind the dye and are detected as blue bands. Ethidium bromide is used to detect DNA in gels as illumination with UV light allows visualisation of the fluorescence of the DNA-bound ethidium bromide. In some cases the molecules are radiolabeled and are detected by autoradiography. The analytes may also be transferred to a membrane and analysed after they have been bound. Examples of this are hybridizations southern, northern, and immunoblotting westerns. These methods are typically time consuming and/or lack sensitivity.

Other methods for assaying analytes of interest are based on the use of filter wicking methods. These are used for various analytes such as glucose, cholesterol, drugs, antigens, antibodies and nucleic acids. In one form, using antibodies, these are called immunochromatographic assays. These test formats using filter strip, wicking methods or lateral-flow offer the beauty of simplicity. All that is necessary is to apply a sample, for example blood, urine or saliva, and this results in the dried and labelled antibody dissolving and mixing with the sample and starting the antibody/analyte binding reaction. During this binding reaction the sample and labelled antibody are wicked (via capillary flow) along a strip which at a predetermined point contains an immobilised second antibody against the analyte of interest. The result is that as the sample and labelled antibody are transported along the membrane or supporting phase, the analyte binds to the antibody. This immune complex then at some point is transported in the liquid flow to the capture antibody which is immobilised at a given site within the filter or supporting phase. When the analyte-labelled antibody complex interacts with the immobilised capture antibody it is captured by binding to an alternative epitope on the analyte of interest resulting in the immobilisation of the label which is attached to the labelled antibody and complexed with the analyte. The fluid continues to wick or flow past the immobilised antibody carrying any un-complexed (not bound to analyte of interest) labelled antibody away from the immobilised capture antibody. At this point, the labelled antibody is detected. These assays use a number of different labelling methods such as particles and enzymes. In other systems based on these wicking or flow methods, additional buffer and/or solutions can be added to give other types of signal and improve the binding reactions. In such a test, the sample is added and allowed to incubate. This is then followed by a wash, and the second binding reagent (labelled) is added followed by a wash. The substrate is then added to detect the bound labelled reagent. These two extremes of the filter wicking, lateral flow or immunochromatographic assay systems are given here to illustrate the potential range of conditions and steps which can make up such rapid assay systems.

Electrochemical-based sensors for the detection of biomolecules have been developed into highly successful commercial products. This has been exemplified by the measurement of glucose based on the detection of electrical changes that occur during an electrochemical reaction. Current glucose detectors use an enzyme such as glucose oxidase to provide the selective recognition and generation of a detectable signal by an electrode. Some of these products, such as the system manufactured by Boehringer Mannheim, use direct electrochemical oxidation of hydrogen peroxide produced by the enzyme. In an alternative system, MediSense Inc. uses a modified ferrocene as a redox mediator between the enzyme and the electrode. This removes problems due to oxygen concentrations and pH (M.G. Boutelle et al. J Mol. Rec. 1996. 9, 383-388). Electrochemical technology has also been applied to the development of electrochemical methods based on the generation of light as a signal (Igen inc, Gaithersburg MD). In this system, a specific label, typically Ru (2,2-bipyridyl)₃²⁺, is used attached to a binding species through a spacer arm and the capture of this label is detected by the generation of excited-state Ru (2,2-bipyridyl)₃²⁺ via a redox reaction at the surface of an electrode connected via an electronic circuit to a porentiostat. The voltage is controlled at the surface of the externally, electronically connected electrodes within the electrochemical cell to surface potentials less than 3 volts due to the nature of the instrument and the electrochemistry of this system. This electrochemistry is conducted using a potentiostat to control the potential at a working electrode. These instruments for controlling electrochemistry are designed to apply voltages of less than 10 volts and electrochemical methods have thus been restricted to these low voltages applied to an externally, electronically connected electrode surface. In an alternative electrochemical system which makes use of light as the detectable signal, luminol and its derivatives can be subjected to electrochemical excitation resulting in the emission of light. The use of the luminol system typically involves combination with a peroxidase-producing enzyme which generated hydrogen peroxide for the activation of the luminol light emission on electrochemical activation.

In summary, there are numerous examples of the use of electrochemistry for assaying biomolecules based on the detection of specific biomolecules directly via electrochemical methods or via the use of enzymes or other biocatalysis used to generate detectable species or via the use of electrochemiluminescent labels as in the case of the ruthenium trisbipyridyl label used by Igen inc, Gaithersburg MD (WO 86/02734). The essential element of all these methods has been the need to bring the reagents and/or binding species in close proximity to an electrode surface which is in direct electronic contact via wires or equivalent electron conduction material to an external control electronic circuit. The electrode systems in these cases are typically made of a three-electrode system with working, counter and reference electrodes in contact with an electrolyte that contains the sample. These three electrodes are then in electronic contact outside the sample or electrolyte chamber via wire or equivalent electron conducting material to a potentiostat. This configuration of electrodes linked via a potentiostat allows precise control of the voltages at the working electrode. In some cases, the electrode configuration is made up of two electrodes and the counter and reference electrode are combined with some success. Thus, all electrochemical-based assay systems have been designed using electrodes that are exposed to an electrolyte containing the sample and connected by external contacts via wire or equivalent electron conducting material to a potentiostat. These electrochemical assay systems make use of measurable electrochemistry, for example, by measuring electronic properties or light generated at the surface of these directly controlled and connected electrodes. These systems suffer from the problem of not being able to detect and analyse the presence of analytes in the bulk solution. If they are able to effectively capture the analytes in bulk solution, e.g. using beads, then these beads need to be captured or otherwise brought to the surface of an externally, electronically connected electrode. This presents a significant impediment to the speed, sensitivity and simplicity of the assay and the instrumentation, for example requiring a magnet (WO 96/15440, WO 92/14139). In the case where the bead is brought to the surface of the electrode, only a small portion of the assay sample is measurable (Williams 1995 IVD Technology November 28-31). In cases where beads are not brought into proximity of the working electrode, actual loss of signal has been described when ECL labels are bound to the bead (WO 90/05301).

Systems involving electrochemical synthesis or production have provided examples of electrochemistry using electrodes consisting of suspended conducting particles. These have been described for the electrolysis of sea water and the production of potassium permanganate (Fleischmann et al US4124453; Agladze et al US4269689; Bharucha et al US3941669). This field of electrochemistry has been called bipolar electrochemistry as each conducting element serves as both anode and cathode ("Applications of Fluidized Beds in Electrochemistry" by P. Le Goff et al., Industrial & Engineering Chem., Vol. 61 No. 10, 1969, pp. 8-17. J.C. Bradley et al. Nature 1997, 389, 268-271). In these experiments, the electrolyte contains a suspension of conducting or semi-conducting particles that are subjected to an electrical field. This results in the generation of anodic and cathodic faces on the conducting particles and results in electrochemical reactions at the surface of the conducting particles. These methods have not been used to generate a detectable signal or material which is related to a binding event or the presence of a molecule or analyte of interest for the purpose of determining the presence or concentration of these molecules or analytes in a sample.

The present invention provides for the application of bipolar electrochemistry in a new way for diagnostic applications and constitutes an improvement over those currently known. Various methods and examples are disclosed.

### Objects and Advantages of the Invention

Accordingly, it is an object of the invention to provide a method for detecting molecules or analytes of interest using a conducting or semi-conducting material - not in electronic connection with an external electronic circuit - as a bipolar electrode for electrochemically generating detectable signals or material. The level of detectable signal or material being detected is related to the amount or presence of the molecules or analytes of interest.

It is another and related object of the invention to describe an apparatus able to detect the signal or material electrochemically generated by the conducting or semi-conducting material which is not in electronic connection with an external electronic circuit.

It is another object of the invention to provide a new label for binding assays which is a conducting or semi-conducting material acting as a bipolar electrode.

Accordingly, the invention is an improvement over the existing art in that it allows the electrochemical reaction to occur more effectively in the bulk solution providing for more rapid assays of greater sensitivity. It is a further improvement in that it provides a means for assaying multiple analytes in an array without the need to provide multiple electrode contacts or large extended electrode surfaces.

### Summary of the Invention

This invention includes an assay method for an analyte of interest using an electrochemical process. The assay comprises the establishment of contact between the sample to be assayed for an analyte of interest and at least one structure of conducting and/or semi-conducting material not in electronic contact with an external electronic circuit; followed by applying a voltage gradient across at least a portion of said at least one structure of conducting and/or semi-conducting material not in electronic contact with an external electronic circuit, said voltage gradient being such that at least one electrochemical reaction takes place on at least one of said at least one structure of conducting and/or semi-conducting material not in electronic contact with an external electronic circuit; then detecting and or quantitating said at least one electrochemical reaction directly and/or indirectly and determining the presence and/or quantity of said analyte of interest.

This invention also includes an apparatus for use in detecting and/or assaying an analyte of interest using electrochemistry, comprising; an electrical power supply able to deliver a voltage; a cell able to accept a sample containing structures of conducting and/or semi-conducting material, wherein said cell contains at least two electrodes which are externally and electronically connected to said electrical power supply and said cell is either an integral part of the apparatus or is a removable element, and a detector able to detect electrochemistry, said detector selected from the following; film, photomultiplier tube, photodiode, phototransitor, CCD camera, electrochemical cell, wherein said detector does not or is not able to detect electrochemistry from said at least two electrodes.

This invention also includes a kit for carrying out an assay comprising; a container with a binding species labelled with an electrochemically-detectable species; a container with at least one structure of conducting and/or semi conducting material coupled with a complementary binding species, wherein said at least one structure of conducting and/or semi conducting material is capable of being stimulated to carry out electrochemistry on application of an applied voltage to an electrolyte in contact with said at least one structure of conducting and/or semi conducting material when not in electronic contact with an external electronic control circuit.

This invention also includes an immunochromatographic device containing;
a binding species labelled with an electrochemically-detectable species,
a binding species immobilised onto a solid phase
and at least one structure of conducting and/or semi-conducting material positioned such that electronic connection to an outside electrical circuit can be avoided.

This invention also includes a method of activating a stabilised dioxetane to emit light which comprises subjecting said dioxetane to electrochemistry resulting in the destabilisation of said stabilised dioxetane to induce its chemical decomposition resulting in light emission.

This invention also includes a method for generating electromagnetic radiation within a separations media which comprises applying a voltage to a separations media containing or constructed from structures of conducting and/or semi-conducting material such that said separations media is not rendered conductive and/or semi-conductive.

### Detailed Description of Invention

This invention relates to electrochemical-based assay systems for analytes of interest such as molecules, ions, amine-containing molecules, antibodies, antigens, nucleic acids, receptors, ligands, hormones, biomolecules and any combination of binding species which, in operation, incorporate various structures of conducting or semi-conducting material. These various structures are not in electronic contact via a wire or equivalent electron conducting media to an external voltage supply. The material of the subject invention is conductive or semi-conductive and acts as an electrode which has both an anodic and cathodic face under an applied electric field. Thus, the material becomes bipolar with respect to the developed electrical potential. The material is in electrical contact via an electrolyte providing an ionic but not electronic contact to an external electronic circuit. The material is stimulated to carry out the desired electrochemical reactions by applying a voltage gradient across at least a portion of the electrolyte which is in contact with the conducting or semi-conducting material. This results in the development of anodic and cathodic portions on the conducting or semi-conducting material surface such that electrochemistry occurs on at least a portion of the anodic or cathodic portions of the conducting or semi-conducting material in contact with the electrolyte. The result of the electrochemical reaction is to produce a detectable signal (such as light) or to produce a detectable molecular species (such as a chromophore or fluorophore or electrochemically-reactive molecule) which allows the detection and/or quantitation of the analytes of interest. The detailed specific methods by which this can be brought about to allow detection and/or quantitation of analytes of interest are further described below.

### Electrical contact and electrical conduction

It is well understood that electrical contact can be established by various methods and can be characterised by the mode of charge transfer. In one method for electrical contact, electrons move to achieve the charge transfer. This form is typically known as electronic. This electronic method of electrical contact is based on electrons as carriers of charge and electricity, as found in metals, conductors or semi-conductors. When an electrolyte is used to create electrical contact and allow electrical conduction, no direct electronic conduction is involved. When an electrolyte is used, the charge transferred during electrical conduction is carried by ionic species (charged atoms and molecules). Thus, electronic conduction is not based on the movement of ionic species as charge carriers. An electrolyte is an ionic conductor capable of forming an electrical contact in all three states of matter: gases, solids and liquids. With electrolytes, charged molecular species move at the interface between an electrolyte and an electronic conductor or semi-conductor where electrochemistry can occur. This electrochemistry is dependent on the applied voltage, chemicals (i.e. electrochemically-active compounds) in the electrolyte and the nature of the electronic conductor or semi-conductor surfaces.

### Conducting and semi-conducting materials

Examples of materials which can be used in this invention to act as electrodes to carry out electrochemistry are without limitation: conducting or semi-conducting materials, composites, mixtures, alloys, blends or other such obvious derivatives which combine many conducting, semi-conducting and non-conducting materials to produce materials which in the final form are able to act as conductors or semi-conductors. Some examples are Si, GaAs, Pt, Ag, Au, C, Zn, Si, Ge, Sn, Pb, Hg, Ti, V, Cr, Mn, Fe, Co, Ni, Cu, Mo, Ru, Rh, Pd, Cd, In, Sb, W, Re, Ta, Os, Ir, Bi, Al, tin oxide, indium/tin oxide (ITO), antimony/tin oxide, electronically-conductive polymers such as polypyrole and polythiophene. These materials may also be used as coatings e.g. carbon, gold, Pt, ITO and the conductive polymers. An example of a coated conductive material is Zelec ECP which is an electronically-conductive powder (DuPont, DE). Zelec is available in two forms, one as a coating of antimony doped tin oxide on an inert core (silica, mica and titanium dioxide) or as solid particles of antimony doped tin oxide. In an advantageous embodiment of the invention C, Au, Pt, ITO and Ag are the most favoured conducting materials of interest in the invention as they present a number of ideal properties including cost, stability, availability and experience with these materials in a number of electrochemical systems. Also, these materials have already been used to manufacture numerous structures, forms and coatings. C is of particular interest because of the number of different forms available and the number of differing electrochemical systems both in electrochemical applications and also in electrochemical assay devices which have made use of this material in its various conducting or semi-conducting forms. Carbon is also readily derivatized to yield numerous specifically modified surfaces: such as in WO 97/33176 and WO 98/12539 which are hereby incorporated by reference. In addition, many other methods are known in the art for modifying carbon and other materials to render them useful for coupling. Examples are cited in the following; US 5,667,667; P Allongue et al J Am Chem Soc 1997, 119, 201; C Sellitti et al Material Science and Eng.1990, A126, (1990) 235-244; C Kozlowski et al J. Chem. Soc., Faraday Trans. 1985, 81 2745-2756; B Barbier J. Electrochem. Soc. 1990, 137, 1757-1764; Au and Pt are also of special interest as these materials are used widely for electrochemistry, electronic fabrication and in immunoassay systems. Au and Pt also have added advantages over many other materials in that they are relatively inert and are stable when exposed to a wide range of chemical agents.

### Structures

Examples of structures of conducting or semi-conducting materials suitable for the subject invention are numerous. These structures can take numerous shapes and forms, such as beads, particles, spheres, spheroids, cylinders, cones, plates, coatings, fibres, fibrils, nanotubes, nanoparticles, colloids, splinters, filings, springs, coils, amorphous particles, amorphous plates or coatings, and as a conductive coating may take the form of two dimensional shapes of squares, triangles, rectangles, circles, pentagons, hexagons, stars etc. In an advantageous embodiment, the structures of most interest will be particles that are spherical or spheres or similar, also fibres, fibrils and nanotubes for the coatings. These may be in the form of conductive coatings on the surface of 3 dimensional structures, but can also be in the form of 2 dimensional shapes such as those produced by the evaporation of gold onto a surface which is masked to produce a desired shape on a surface. Other examples of coating methods are without limitations, e.g. sputtering, screen printing, plating, polymerisation, stamping and painting. Many materials can be coated onto surfaces of flat or shaped structures thus producing a desired conducting or semi-conducting surface to form a structure for the purposes of the invention.

In the cases of evaporated gold, sputtered carbon shapes or pyrolysed carbon on a surface, the potential exists to generate an array of such gold or carbon elements where each may function as separate bipolar electrodes as described for this invention. Other coating methods, as illustrated above, could also be used in this way to generate a series of potential bipolar electrodes. The present invention using arrays of patterned conducting elements does not need to be in electronic contact with an outside circuit. As a result, the invention solves a problem inherent to the application of multiple electrodes or multiarray electrodes used in the development of electrochemical assay systems for multiple samples and multiple analyte detection systems.

In the case of particles or beads, it is understood that particles or beads from cm to nm in size have been used in immunoassay applications ranging from tube-based assays to filter wicking assay systems. We anticipate the use of particles in these ranges as structures of the subject invention.

It is also anticipated that the structures of conducting and/or semi-conducting material may also be incorporated into composite materials by mixing with non-conducting and/or conducting and/or semi-conducting materials. By way of an example, filters may be constructed by the inclusion of structures of conducting and/or semi-conducting material during manufacture to generate a non-conducting filter material which contains dispersed structures of conducting and/or semi-conducting materials. These composite filters are valuable in the development of the immunochromatography methods of the invention. When wetted with electrolyte, these composite filters can be activated to carry out electrochemistry through the bulk of the filter at the sites of the dispersed structures of conducting and/or semi-conducting material. In other forms of composites the concentration of the conduction and/or semi-conducting material is such that the composite is itself conducting and/or semi-conducting. The composites of this type are then equivalent to the conducting and/or semi-conducting materials of the subject invention. These composites can then be used to form the structures of the invention as described above forming for example fibres, particles, beads etc. Other types of composite containing conductive and semi-conductive structures of the subject invention are gels and sols and hydrogels. In these composites with gels, sols and hydrogels, the conductive material is dispersed or suspended in such a way that the conductive or semi-conductive structures do not render the composite electronically conducting. In addition, the amount of conductive or semi-conductive structures is such that the final form of the composite is visible to various electromagnetic radiation and photon detection means. Also, such composites are amenable to the use of conventional electrochemical detection methods using electrochemical cells externally connected to a electronic circuit.

Some examples of materials which are readily available and illustrate some of the forms contemplated in this invention are: Aluminium powder, 20 micron; Beryllium powder, 10-20 micron; glassy carbon spherical powder in various sizes from 0.2 µm to 2,000 µm; graphite powders; graphite fibre 8 µm in diameter and 6-25 mm long; gold powder, spherical, 2-8 µm; gold flake, 2-15 µm; nickel powders, 2-7 micron, various forms; palladium powder, various forms from 0.25-7.5 micron; platinum powder, various forms, 0.2-3 micron (Alfa Aesar, Ward Hill, MA); carbon nanotubes or fibrils (Materials and Electrochemical Research Corp, Tucson, AZ); Zelec ECP (DuPont, DE).

In addition, the fabrication of the structures from the materials of the invention is highly developed allowing a wide range of shapes and structures as outlined above. For example, so-called nanowires which can be up to 55 µm long and about 250 nm in diameter have been made (Huber, CA, Science 1994, 263, p800). It is contemplated that nanowires of various sizes and material can be used advantageously in the present invention as structures of the subject invention. It is also contemplated that structures of the subject invention, as described above, may be coated to improve the electrochemistry or reduce interferences, for instance with gels, polymer layers or grafts and solid electrolytes layers which are known in the art.

### Detectable electrochemical reactions

Detectable electrochemical reactions consist of electrochemistry on a species (a so-called electrochemically-detectable species) which gives rise to a directly detectable species such as photons of electromagnetic radiation or indirectly via production of a subsequently-detectable species or molecule such as a chemiluminescent, fluorescent or coloured molecule or a molecule with an altered absorption spectrum, and electrochemically active species.

Numerous examples of detectable electrochemical reactions exist and produce electromagnetic radiation, electrochemically active molecules, chromogenic molecules and fluorogenic molecules. Advantageously, the detectable reaction is directly coupled (i.e. comprising typically no more than four to five reaction steps) to the electrochemical reaction at the electrode surface but may also be indirectly coupled to the reactions at the electrode surface where numerous reactions linked to an initial electrode reaction result in the production of a detectable signal, species and/or molecule.

The electrochemical reactions that produce electromagnetic radiation, such as ECL, chemiluminescence, and electroluminescence, are easy to detect as they require simply a means to detect the electromagnetic radiation produced. In the case of ECL and chemiluminescence, the electromagnetic radiation is produced from electrochemical reactions either directly or by coupling to other electrochemically-produced reactants. Various forms of ECL are considered in this invention, for example, ECL from Luminol with hydrogen peroxide, ECL from metal chelates and organic molecules which can occur in the presence of co-reactants such as oxalate (D Ege et al, 1984, Anal Chem 56, 2413-2417) and numerous amines as described in WO 97/33176 and EP 0441894B1 which are incorporated by reference in their entirety. Also, the ECL from Luminol with hydroperoxides, e.g. in the measurement of lipid hydroperoxides (A. Zamburlino, Biochim Biophys Acta (1995) 1256, 233-240). ECL may also be generated from a number of metal chelates and organic molecules where use is made of the electrochemically-generated oxidised and reduced species (generated at the anode(s) and cathode(s)) to produce the excited state for light emission. Light emission may also be activated electrochemically from other chemiluminescent molecules in addition to luminol such as acridinium ester and advantageously from stabilised dioxetanes such as those sold by Tropix (Waltham, MA). In the case of the stabilised dioxetanes, these have typically been stabilised by the use of substituent groups which can be removed enzymatically to destabilise the dioxetane and thus produce light. In the subject invention, the dioxetanes may be stabilised by groups which are or can be electrochemically removable providing for a wider range of potential chemistries; examples of such groups are alcohols, acids, and glycosides (VG Mairanovsky, 1976, Angew. Chem. Int. Ed. Engl. 15, 281-292).

It is also contemplated that fluorescence would be used as a detectable signal or label. Fluorescence could be used either via the synthesis or the activation of a fluorescent species. For example, the local pH changes brought about in the proximity of a micro electrode can be sensed by the fluorescein activation caused by the pH change in the local environment of the electrochemical reaction (BB Ratcliff et al 1996, Anal Chem 68, 2010-214). An electrogenerated fluorescent species may also be detected as demonstrated by McLeod et al, (The Analyst, 1982, 107, 1-11).

In another example, the electrochemical reaction, which is detectable, allows the detection of the conducting and/or semi-conducting structure. In this example, the conducting and/or semi-conducting structure becomes the detectable species and can be used to detect the analyte of interest. This property has been described for Al which can emit light on application of a voltage at anodic and cathodic surfaces (Haapakka K et al, 1988, Anal Chim Acta 207, 195-210).

A conducting and/or semi conducting structure may also be coupled to a binding species to form the electrochemically-detectable species. When bound or captured, this could subsequently be detected using electrolytes with ECL and CL species such as Ru(bipy), luminol. The capture of a structure of the subject invention would allow for considerable enhancement of the signal as a single binding event would provide a bipolar electrode which could generate more light in an ECL reaction than from the capture of a few ECL active molecules. The conducting and/or semi conducting structure may be made from or coated with electroluminescent material and in this form no additional chemistry would be needed to support the generation of light from structures in the electrolyte when a suitable voltage is applied.

### ECL labels

The ECL labels of the subject invention include numerous examples of both organic compounds and metal ion chelates. Well known examples are Ru tribipy, Ru tris phen, Os tribipy, and numerous other metal chelates described in the literature, and luminol. Numerous examples are covered in the following publications: US5453356, US5591581, US5238808, US5221605, WO 97/33176, WO 96/28558 which are incorporated by reference in their entirety. Also, Al structures are known to luminesce when coupled to an electrochemical reaction in an electrolyte (Haapakka K et al, 1988, Anal Chim Acta 207, 195-210).

### Electrolytes

The electrolyte of the present invention typically contains ionic species in solutions, solids and gases to allow electrical conducting. A charge passing through an electrolyte is transferred via movement of the ionic species in the bulk solution, solid or gas. In addition to these basic elements, in many cases the ionic species is also involved in the control of the ionic strength and/or the pH of the electrolyte such that it is optimal for the assay of the analyte of interest and/or the electrochemistry. Additionally, it is common to find within the electrolyte the addition of coreactants used to enhance or support the electrochemistry of the detection system. An example of an electrolyte that can be used for ECL with Ru (2,2-bipyridyl)₃²⁺ is phosphate buffer used as electrolyte and pH control, combined with tripropylamine as an amine coreactant to support the ECL electrochemistry.

Numerous examples of buffers exist, for example borate, phosphate, tris, HEPES, MES, MOPS, PIPES, acetate, triethylamine, tripropylamine, bicarbonate with the appropriate counter ion. In some cases, the buffer may also be a coreactant e.g. amine-containing buffers can act as coreactants in Ru (2,2-bipyridyl)₃²⁺ -based ECL.

The ionic strength of the medium can be controlled typically by adding salts, but may be established by controlling the concentrations of the buffer and/or the coreactants. Examples of salts which may be used to control the ionic strength are numerous. Examples are salts of K, Na, Ca, ammonium, guanidinium.

Typically, electrolytes of the subject invention are based on water solutions, but organic solvents may be used and in some cases advantageously during the electrochemical reaction. It will be understood that mixed solvents can also be used advantageously. For example, formamide is used advantageously in DNA probe applications.

Other components may also be added to improve assay conditions. Typical examples of components used to improve immunoassays are detergents, other proteins and lipid components. These other components are known in the art and are typically optimised for various assays under the various conditions of differing assay systems. The optimisation of these components is well known in the art and forms an integral part of assay optimisations. The use of other components to enhance various aspects of nucleic acid probes is also known in the art.

### Instruments

Basically, the instruments integrate a luminometer, a voltage control circuit and a cell with two externally electronically connected electrodes. Optionally, the apparatus may include a pump or other fluid-handling components and a flow-through cell with externally electronically connected electrodes.

The instruments of the subject invention advantageously have a cell which contains at least one electrolyte. This cell contains at least two electrodes which are in electronic contact with an external electrical circuit which is able to apply a voltage. These two electrodes are in contact within the cell via the electrolyte within the cell. It is possible to design cells of various shapes and configurations such as flow-through cells, single-chamber cells which allow the introduction of electrolyte at desired times. It is also possible that the cell and electrodes are a single module which can be inserted into the instrument to make contact. The externally electronically connected electrodes may be isolated from a central zone or at least from each other by a membrane, gel or other material which is semi permeable to ions and thus maintains contact between the two externally electronically connected electrodes via the electrolyte. This use of a semi permeable membrane may be valuable for isolating the sample from the externally electronically connected electrodes and from the electrolyte in which they are immersed. It may also be valuable to prevent both the detectors and the sample from being affected by the electrochemistry occurring on the externally electronically connected electrodes.

Ideally, the various instruments contemplated would be controlled by a microprocessor either directly by incorporation of a microprocessor into the instrument and/or by an external microprocessor such as a computer. Software for controlling such interfaces is well known to those in the art and may also be written for custom applications to make use of internal and external microprocessor control e.g. Labview from National Instruments (Austin Tx) www.natinst.com. In addition it will be understood that simple electronic circuits can be constructed to control the application of voltage to the cell. Thus, the control of such an instrument can be achieved at various levels from simple mechanical systems to microprocessor controlled systems.

An example of an instrument of the subject invention contains a cell which is able to contain an electrolyte of interest for the detection and/or quantitation of an analyte(s) of interest which further consists of three zones or functionally distinct areas. Two of the zones flank a central zone and contain externally (to the cell) electronically connected electrodes. These two externally electronically connected electrodes are used to apply voltages to the central zone of the cell. The two externally electronically connected electrodes are externally connected to a controlled voltage source. Typically, this controlled voltage source is under the control of a computer but may be controlled by other means such as a simple electrical circuit or mechanical means for triggering the application of the applied voltage. The controlled voltage applied to at least the central zone is initiated by an operator or by determining the presence of, or placing, the sample electrolytes in the central zone of the cell. The sample electrolyte contains the conducting and/or semi-conducting structures of the subject invention along with the compound(s) which are able electrochemically to produce a detectable species allowing detection of analytes. The two externally electronically connected electrodes electronically connected to the controlled voltage source are thus able to apply a desired voltage gradient to the central zone of the cell controlled by electronic means including a simple electronic circuit and/or microprocessor. The application of this desired voltage to the central zone of the cell allows the conducting and/or semi-conducting structures of the subject invention to carry out electrochemistry. This desired electrochemistry results in the formation directly or indirectly of a detectable species which when detected allows the detection and/or quantitation of the analyte of interest.

The central zone of the cell is fashioned in such a way that it readily allows the detection of the detectable species generated by the electrochemistry. In this regard, the central zone will contain at least one window. This window will permit the detection of electromagnetic radiation from the central zone. In an alternative embodiment, the window is present and additional externally and electronically connected electrodes are introduced through the open window into the central zone, or into a zone in a flowpath from the central zone, to allow electrochemical detection of the detectable species using conventional and understood electrode configurations in combination with conventional electrochemical methods such as amperometric or potentiometric methods. Examples of these are glucose and oxygen sensors and oxygen electrodes.

In the case of ECL and chemiluminescence, the window will permit photon detection by a means of choice to detect and/or quantitate the photons from the central zone.

In the case of a chromogenic detectable species, the central zone would allow a beam of photons to pass through the sample in the central zone and be analysed and/or detected and/or quantitated. The analysis of this beam of photons after passing through the sample in the central zone would determine whether the intensity of the photon beam had changed and/or if the spectral properties of the photon beam had been changed. In an advantageous embodiment, the beam of photons would have a wavelength selected to interact optimally with the detectable chromogenic species of interest.

In the case of a fluorogenic detectable species, the central zone would allow a beam of photons to pass through the sample and be analysed and/or detected and/or quantitated. The analysis of this beam of photons, after passing through the sample in the central zone, would determine the number and/or intensity of photons emitted from the sample in the central zone and would determine whether the spectrum had changed with respect to the incident beam of photons. In an advantageous embodiment, the beam of photons would have a wavelength selected to interact optimally with the detectable fluorogenic species of interest and the detector would be optimised to detect and/or quantitate the emitted photons from the detectable fluorogenic species. The use of time-resolved fluorescence is also a contemplated detection means. The use of fluorescence and/or other energy transfers is also contemplated as a means of signal enhancement and discrimination, as used in other assay systems known in the art. Other detection means are also contemplated by this assay system and instrument, such as electrochemical means for the products of the electrochemical reactions at the surface of the structures of the subject invention.

### Cells of the instrument

Various cells for the instrument of the subject invention are contemplated and it is understood that numerous potential examples are possible. Examples are described in detail in the section "Examples".

In the case for an instrument able to analyse clinical samples, a flow-through cell with the three zones described above would be advantageous.

When using the subject invention with rapid immunochromatography-based assays, a cell would be a section of such a device. This section could be removable and is potentially a disposable element.

In a multiwell plate, the structures of the invention could be coated onto the surface of the well in a microtitre plate, typically on the bottom or portions of this part of the well, either as a single contiguous conducting or semi-conducting structure or advantageously as multiple elements in the size range of 1 µm to 1 mm as measured between the edges of the individual structures in the plane between the externally electronically connected electrodes. The wells in this multiwell format could have the externally electronically connected electrodes within the well or these could be introduced at a suitable time to apply the electrical field needed to render the conducting or semi-conducting structures bipolar thus stimulating electrochemistry. This would result in the production of a detectable signal. The multiwell plate may also be constructed using composite materials containing conducting and/or semiconducting structures of the subject invention. Multiwell plates so constructed would possess well surfaces of conducting and/or semiconducting structures able to act as bipolar electrodes in an applied field.

It will also be understood that the structures of the invention as suspended structures in assay buffers may be used in a multiwell plate format in addition to the potential to include them in the plate structure.

In a multianalyte system, the conducting or semi-conducting structures of the subject invention could take the form of multiple individual patterns or shapes on the surface of a non-conducting material forming a multitude of potential assay sites rendered bipolar in an applied electrical field. These bipolar structures of the subject invention would then be capable of electrochemistry in electrolytes known to the art. The result of this electrochemistry would be to allow production of a detectable signal dependent on the presence of an electrochemically active species in proximity to the bipolar structures of the subject invention. In this multianalyte cell, the surface with its multiple individual patterns or shapes of conducting or semi-conducting material may form the bottom of a well in an assay plate with many other such surfaces as found in a microtitre plate or be part of a flow-through system as a means for introduction of sample and or reagents. In these and other related cells, the surface with its multiple structures must be in contact with an electrolyte and must have at least one window to allow the detection of the detectable species. This provides for significant advantages over the current art which requires external electronic connections to each element. Further examples of cells are described in the section "Assay formats" and the section "Examples".

### Waveforms and voltages

During analysis of the sample, a voltage is applied to the sample. The following section describes some examples of this and the waveforms the applied voltage may take.

Applied voltage profiles can be those seen in typical electrochemical studies and cells with the exception that these are not limited to the low voltages seen in normal electrochemical cells (less than 5 volts). The voltages which may be applied to the structures of the subject invention range from 2 volts/cm to 50,000 volts/cm or the limit of the solvent/electrolyte used. Advantageously, voltages range from 20 volts/cm to 50,000 volts/cm or the limit of the solvent/electrolyte used. Most advantageously, the voltages range from 100 volts/cm to 50,000 volts/cm or the limit of the solvent/electrolyte used. The voltage applied to the cell of the subject invention via the externally and electronically connected electrodes can take multiple forms. For example, the voltage may be applied in a step form where the potential is raised to a given value and held for a time and then removed. This type of step may be applied repeatedly to give a square wave form with a wide range of frequencies from 0.001-10,000 cycles per sec (advantageously this is from 0.01 to 1,000 cycles per sec). The voltage may also contain portions which apply reverse potentials. Thus the step potential may be described as a negative and then a positive portion and this single unit may be repeated to give an alternating square wave form. The voltage may also be applied in such a way that it follows a ramp up and down in voltage with or without negative or reverse polarity portions to the ramp. These ramps may be constructed around numerous potential waveforms such as sin waves, saw tooth waves, multiple component step waves and combinations of these. Also, it may be advantageous to have at least a lower voltage portion in such wave forms prior to the excitation of the electrochemically detectable species. Where a single cycle of applied voltages is used, this cycle may take from 0.01 to 1600 secs to complete, advantageously this may be from 1 to 60 seconds.

### Measuring cycle

The measuring cycle in the instrument consists of the introduction of the sample into the central zone of the cell either by flow (in a flow-through system), by robot arm or other manual or mechanical pipetting means, or by placing a cartridge and/or disposable element containing the electrodes, conducting and/or semi-conducting structures and sample into the instrument. The sample is then subjected to a voltage waveform or pulse as outlined above and the detectable electrochemical reaction is then detected.

The sample may be subjected to sonics or ultrasonics to improve the kinetics of the binding reaction and/or the electrochemistry as has been described previously for these reactions and is known in the art (K Suslick, Science, 1990, 247, 1439-1445 and D Walton et al Electrochimica Acta, 1993, 38, 307-310). Mixing may also be achieved via the use of magnetic methods or other mechanical methods known in the art which are known to achieve significant fluid mixing. These various forms of mixing can be advantageous in that the mixing during the application of the applied voltage, activating the electrochemistry, can induce tumbling and rotation of the structures of the subject invention is such a way that significantly more surface of the structures is activated to carry out the desired detectable electrochemical reaction(s).

The voltages and waveforms described above may also be applied during mixing or sonication or ultrasonication. In addition, multiple (advantageously from 2 to 20) externally and electronically connected electrodes may be used in a cell to achieve the application of a voltage field at various angles with or without the application of alternating positive and negative fields to achieve activation of a greater surface of the structures of the subject invention. In one of its forms, externally and electronically connected electrodes could be positioned at the eight positions of a sphere, or corners of a cube or rectangle defining the central zone of the cell. These electrodes could then be used in pairs or other various combinations to apply both negative and positive voltages to the cell. These voltages could be controlled by a computer or mechanical device to apply a sequence of voltages to the different electrodes in such a way that all faces of the conducting and/or semi-conducting material are activated to carry out the desired electrochemistry to produce the detectable signal. Activation of various faces of the conducting and/or semi conducting structures of the subject invention could also be used in combination with multiple detectors of electromagnetic radiation to permit correlation of applied voltages and electromagnetic radiation from various faces to be made thus allowing a more effective determination and elimination of background signals in the system and from the detectors and their associated circuits.

The various forms in which the electrochemically-detectable signal can be detected are numerous and are known in the art, for example: in the case of ECL and chemiluminescence, a light detection method based on the use of photomultiplier tubes and photodiodes (luminometers), CCD cameras, film and by eye; in the case of a fluorescent molecule generated by the electrochemistry, a detector based on a fluorimeter would be used; in the case of a chromogenic or other molecule with a new absorption spectrum, a spectrophotometer system would be used; in the case of an electrochemically active molecule generated as the electrochemically detectable signal, an electrochemical cell with externally electronically connected electrodes would be used to electrochemically detect the species generated using potentiometric or amperometric methods.

### Binding Species

Various assays are described for the subject invention and these can be carried out making use of numerous binding species. The following are examples illustrative of the types and kind of binding species contemplated in the subject invention. In this discussion, a molecular species includes chemicals from single species atoms or ions (i.e. Ca²⁺) to complex multiple atom compounds (i.e. antibodies). Most binding species are defined by the existence of a partner binding species since, to be a binding species, the molecular species must bind to another molecular species which may be the same molecular species. For example, an antibody may be developed such that it will bind to itself. In many cases, this type of self binding is not valuable but can be useful in certain cases, for example, where development of signal amplification is of interest. In the case of a binding species and its binding partner, these two species can be considered to be binding species since they are both able to bind to another molecular species. For example, in the case of antibodies and antigens (well known binding species), the antibody binding species' partner is the antigen and the antigen binding species' partner is the antibody. Thus, the antibody and the antigen can both be considered to be binding species. The following is a partial list of such binding species contemplated in the subject invention: antibodies, antigens, receptors, enzymes, enzyme inhibitors, drugs, hormones, ligands, receptor ligands, lectins, selectins, integrins, RNA, DNA, nucleic acid analogues, nucleic acid binding proteins, chelating ligands. These binding species are all contemplated as analytes that can be detected and/or quantitated by the subject invention.

### Analytes

Analytes of the subject invention are numerous, including the binding species described above, but also including molecules which may not be binding species or can be detected without being part of a binding-species to binding-species interaction. The character which defines these groups is their ability to interact with the electrochemistry in a detectable manner, this interaction occurring because of the applied voltage on the structures of conducting and/or semi-conducting materials not in external electronic contact. Also considered as analytes are molecules able directly or indirectly to produce, destroy or metabolise electrochemically detectable species. An example would be the detection and/or quantitation of amine-containing compounds which act as a coreactant in the generation of ECL from various metal chelates such as Ru(bpy)₃²⁺. Examples of this are contained in EP 0 441 894 B1 which is incorporated by reference. Other examples are tripropylamine, NAD(P)H, and hydrolysed beta lactams. It will be understood that ECL labels (as described earlier) and ECL active species such as Ru(bpy)₃²⁺, ruberene, luminol, and dioxetanes are also considered as analytes. No binding interactions occur in this reaction. Also, in the case of glucose detection and/or quantitation, where glucose is acted upon by glucose oxidase, the product of this reaction (hydrogen peroxides) act as a coreactant with luminol to electrochemically generate light. It would be understood from such a reaction that in addition to glucose as an analyte, glucose oxidase and hydrogen peroxide could also be analytes. It will be understood that an analyte may be subjected to a series of reactions giving rise to a molecular species which can directly participate in modifying (enhancing or inhibiting) a detectable electrochemical reaction. An example of this is seen in the case of a glucose assay based on a well understood assay for glucose which first kinases the glucose (with ATP and the enzyme hexose kinase) to form glucose-6-phosphate. This is then subjected to the action of glucose-6-phosphate dehydrogenase in the presence of NAD⁺ to generate NADH. This acts as a coreactant with Ru(bpy)₃²⁺ to generate ECL (Jameison F, et al. 1996, Anal Chem 68, 1298). Assays based on coupling multiple reactions and/or chemical reactions such as that described for glucose are known and well understood and those reactants, enzymes and substrates which participate to give rise to an electrochemically detectable reaction can be considered as analytes. Thus, it will be understood that many other chemicals may be analytes in addition to the binding species described above. Some example of analytes include enzyme substrates, enzyme products, enzymes, whole cells, viruses, sub cellular particles, nucleic acids, polysaccharides, proteins, glycoproteins, lipoproteins, lipopoylysaccharides, lipids, fatty acids, peptides, cellular metabolites, hormones, pharmacological agents, tranquillisers, steroids, vitamins, amino acids, sugars, and non biological polymers.

### Assay formats

Numerous assay formats are contemplated and are given in the form of examples below.

The assay formats of the subject invention provide various advantages over the current assay systems. Also provided are new ways of carrying out analyses which allow for a series of contemplated improvements to assay systems and scientific exploration.

One key advantage over the current electrochemically based detection or assay systems - where the electrochemistry is confined to the surface of an externally electronically connected electrode - is that the subject invention does not present any such limitation. The subject invention allows for electrochemical stimulation within a sample volume, generating a detectable signal which allows for the detection and/or quantitation of analytes within the sample volume and to a greater extent than previously possible because of the effective extension of the electrode surface area available. This ability to generate a detectable signal by stimulation of electrochemistry within the volume of a sample provides the following advantages :
1. Improved detectability, more of the sample is activated,
2. Improved sensitivity, more signal is developed as the sample volume is significantly larger than in conventional electrochemical methods,
3. Improved speed, improved kinetics and time to analysis,
4. Increased volumes for samples allows activation through the sample volume,
5. Spatial discrimination, allows multiple samples to be analysed and provides the potential of photographic-like resolution within a sample i.e. a gel. This spatial discrimination can also be three dimensional,
6. Improved electrochemistry via redox cycling especially when structures of the subject invention are coupled to or linked via a binding interaction which is mediated by an analyte of interest, thus allowing detection of the binding reaction without the need for separations or washes following the binding interaction. Assays in separation media.

These advantages of the subject invention are significant when determining the location of labelled material localised within a large volume or surface. Examples of this are electrophoretic separations, chromatographic separations (TLC, HPLC, FPLC), immunochromatography, southern blots, western blots, northern blots. In the case of electrophoretic separations, where the detection of the positions and amounts of each species within the gel is of interest, various methods are used for detection and quantitation, i.e. staining with various dyes and fluorescence. Typically, this results in staining the gel directly with various agents to visualise the molecules in the gel, e.g. using commassie blue to detect proteins in gels. Here, the proteins bind the dye and are detected as blue bands. Ethidium bromide is also used to detect DNA in gels followed by illumination with UV light to visualise the fluorescence of the DNA-bound ethidium bromide. In some cases, the molecules are radiolabeled and are detected by autoradiography. In the case of this invention, it is possible to manufacture gels which contain structures (i.e. particles, fibres, fibrils etc.) of conducting material which can be activated to carry out electrochemistry to allow the detection of analytes separated by the gel electrophoresis. Numerous examples of gels, papers and other electrophoretic supports are known in the art e.g. polyacrylamide, agarose, starch, cellulose acetate, cellulose, silica, nylon, nitrocellulose, PVDF, etc.

In an advantageous embodiment of this gel detection application of the invention, analysis of PCR products could be carried out as follows. Oligonucleotide primers are labelled with an electrochemiluminescent (ECL) label ORIGEN TAG (IGEN, Gaithersburg MD) following methods described by the manufacturer (application notes describing PCR are available on the internet at http://www.igen.com). These PCR primers are then used in a PCR to amplify and generate an ECL-labelled PCR product which is dependent on the presence of DNA complementary to the labelled PCR primers. The PCR product can now be run on a standard agarose or polyacrylamide gel containing for example advantageously carbon nanotubes (Materials and Electrochemical Research Corp, Tucson, AZ) as conducting and/or semi conducting material. These nanotubes would be included at such a concentration that the gel is not conductive and is not totally opaque such that no ECL would be visible, advantageously 0.1-10 µg/ml. It is understood that other suitable conducting or semi-conducting material (as described above) may be used in the gel. The gel is then loaded with the PCR products and the DNA subjected to electrophoresis at voltage gradients typical for the separation of PCR products i.e. less than 100v/cm. After the electrophoretic separation, the gel is then soaked in ORIGEN assay buffer (IGEN, Gaithersburg, MD) for 30-60 min with three changes of buffer. This gel is then placed in a high voltage chamber contained within a black box to exclude as much light as possible with electrolyte in contact at each end of the gel as in a typical electrophoresis chamber. The high voltage container has an electrode at each end as is typical of an electrophoresis chamber with the gel between these electrodes. The gel is visualised by a photomultiplier tube, film or CCD camera. The door to the black box containing the gel, high voltage chamber and photon detection system (PMT(s) or CCD camera or photodiode(s)) is closed to create a substantially light-free environment. The high voltage chamber is then subjected to a high voltage which typically increases at a rate of 100 v/s to 10 kv/s but could also be in the range of 10v/s to 1,000 kv/s. This voltage ramp is applied for 0.1 sec or longer. During the application of these high voltages, peak voltages are controlled to prevent arcing which would indicate an excessive voltage. The voltages reached are advantageously from 100 v/cm to 10,000 v/cm. During the high voltage sweep the light produced is detected by the CCD camera (or other light detection means) and the location and/or amount of the PCR product is determined.

In a further embodiment of the above protocol, the electrophoresis buffer would also be the ECL buffer as in the case of a buffer (as described earlier) which contains amines useful for the generation of ECL, for example tripropylamine-phosphate, tripropylamine-acetate, triethylamine-borate, tripropylamine-borate, PIPES and HEPES (WO 97/33176). Commercial examples of ECL buffers are ProCell (Boehringer Mannheim) and ORIGEN assay buffer (IGEN). Alternative methods for ECL could also be used where different pHs may be desired for electrophoresis, for example by the use of the tris(2,2-bipyridyl) ruthenium oxalate system. In this case, where the ECL enabling buffer and/or co-reactants are present, the gel could be pulsed to the higher voltages at desired times during the lower voltages used for the electrophoretic separations to activate the ECL labels on the labelled molecules. The higher voltages are required to render the carbon nanotubes or fibrils bipolar (generating an anodic and cathodic face on at least some of the carbon nanotubes or fibrils, or other structures of conducting and/or semi conducting material) resulting in the activation of the ECL label contained in the PCR products separated on the gel, as described above, or other labelled molecules. This high voltage pulse used to render the carbon nanotubes or fibrils bipolar and activate the electrochemistry on part of the surface of at least some of the carbon nanotubes or fibrils could thus be part of the electrophoresis protocol. This would allow the electrophoretic migration to be followed over time by intermittent activation of the ECL labels attached to the labelled PCR products. This *in situ* monitoring of the electrophoresis would be valuable, improving the speed and resolution of the electrophoresis. These electrophoretic methods, of great interest for the analysis of biomolecules, could also be applied in the analysis and detection of ECL labels not coupled to biomolecules. The application of this method to the analysis and detection of ECL labels and ECL coreactants not coupled to biomolecules could also be extended to ECL active compounds such as Ru (bpy)₃²⁺ and to compounds able to stimulate, enhance and support ECL, such as tripropylamine.

A further modification of the above example concerning the *in situ* detection of an analyte of interest within gels or other separation media is also contemplated. This method contemplates the detection of numerous molecules able to support ECL as has been demonstrated for numerous amine-containing molecules such as TPA, TEA, PIPES, HEPES, beta-lactams, NADH, and enzymes coupled to NAD or NADP etc. This provides the potential to detect enzymes and substrates of enzymes either directly or indirectly via a series of enzyme reaction steps (Martin AF et al, 1997, Biosensors and Bioelectronics 12, 479-489, GA Forbes et al Analytica Chimica Acta 347, (1997) 289-293). This is also possible with enzymes that give rise to hydrogen peroxide, e.g. glucose oxidase, choline oxidase etc., the resultant hydrogen peroxide being detected via coupling to the ECL of luminol. This luminol ECL can also be coupled to the detection of hydroperoxides such as lipid hydroperoxides. In this format, the conductive or semi-conductive material may be precoated with an ECL active label sensitive to the presence of amines or other potential coreactants that enhance ECL, e.g. Ru (2,2-bipyridyl)₃²⁺ when these amines or other potential co-reactants are in close proximity to the dispersed conducting or semi-conducting material coated with the ECL active species. This type of detector system would be useful in the detection of materials in gels, material eluting from columns during purification, during capillary electrophoresis and obviate the problems with the application of conventional electrochemical stimulation which requires sample within a narrow region of an externally electronically connected electrode surface which, being able to analyse or activate only a very small portion of any given sample, results in poor sensitivity for these conventional methods. Here, the coated conducting or semi-conducting material would be excited by the application of an external voltage to generate bipolar faces on at least some of the dispersed conducting or semi-conducting material, and thereby allow the detection of the ECL co-reactants such as amines, beta-lactams, NADH, NADPH and peroxides. In a further variation of this method, the ECL active species may be added in solution i.e. in the gel, HPLC or capillary electrophoresis buffers. This would remove the need to immobilise the ECL species to the conducting and/or semi-conducting structures of the subject invention. Alternatively, this system could be used to detect ECL labels directly or by supporting ECL with a co-reactant. In the case of the luminol peroxide system, peroxides could be detected e.g. hydrogen peroxide or lipid hydroperoxides.

In an alternative embodiment of the above example, these conducting or semi-conducting materials coated with an ECL active species sensitive to co-reactants such as amines, peroxides, beta-lactams, NADH and NADPH, could be used as suspended electrode systems for the detection of various molecules which affect the concentration of these co-reactants. For example, enzymes acting on various substrates can affect the concentration of these co-reactants allowing detection of either the enzyme or the substrates of the enzyme. An example would be dehydrogenases that generate NADH or NADPH, e.g. glucose-6-phosphate dehydrogenase which can be used in a glucose assay when coupled to hexokinase as described earlier. In this system, the analyte of interest is a molecule which can be specifically acted on by a dehydrogenase to produce NADH or NADPH. This is then detected and quantitated by the activation of ECL. In the previous example this is glucose-6-phosphate. The assay could be carried out as follows: the sample would be mixed with the appropriate dehydrogenase and the appropriate co-factor such as NAD or NADP. This enzyme/substrate/co-factor mix would then be supplemented with particles, fibres or fibrils of conductive or semi-conductive material precoated with an ECL active label sensitive to the presence of NADH or NADPH, e.g. Ru (2,2-bipyridyl)₃²⁺. Alternatively, the conductive or semi-conductive material could be added with an ECL active species such as Ru (2,2-bipyridyl)₃²⁺ free in solution. After the conductive or semi-conductive material and the ECL active species sensitive to the NADH or NADPH co-reactant have been added, a voltage is applied. This results in the production of bi-polar faces on the conductive or semi-conductive material leading to ECL which is dependent on the concentration of the analyte of interest. In addition to the potential to immobilise the ECL active species, it is also possible to immobilise additionally the enzyme and the co-factor. These systems - using conventional electrochemical methods - have been described by Martin AF et al, (1997, Biosensors and Bioelectronics 12, 479-489). It is also possible to add the ECL active species into the solution, not immobilised on the conducting or semi-conducting structures of the subject invention.

These examples of embodiments of the invention concerning the detection of molecules within a large volume of sample (not possible with current electrochemical detection methods) are directed at ECL but could also make use of other detectable signal systems such as fluorescence, chemiluminescence or spectrometric methods as described in other sections of the invention. These alternative detectable signal systems are activated by the EC that itself is induced by the application of elevated voltages, inducing the appropriate bipolar character on the conducting or semi-conducting materials within the bulk or volume of the sample.

Assays using filter wicking and/or immunochromatography

In an advantageous embodiment of the subject invention, the detection of antigens, antibodies, receptors, ligands and nucleic acids and their analogues is possible. This embodiment relates to an immunoassay, nucleic acid assay or other similar binding assays carried out using a filter strip or wicking method and also called immunochromatographic tests or lateral-flow tests. Examples of these are found in US 5591645, US 4861711, EP 0 258 963 A2, US 4703017 and US 5008080 which are hereby incorporated in entirety by reference. This embodiment relates to the electrochemical activation of at least part of such a filter or flow-supporting material such that an analyte contained within or bound throughout the body of the filter or flow-supporting material is detected and or quantitated. These tests using a filter strip or wicking method and also called immunochromatographic tests or lateral-flow tests, offer the beauty of simplicity. They involve simply the application of a sample (e.g. blood, urine, saliva). The application of the sample results in the dried labelled antibody and the other assay reagents dissolving and mixing with the sample to start the antibody-analyte binding reaction. During this binding reaction, the sample and labelled antibody is wicked (via capillary flow) along a strip which, at a predetermined point, contains immobilised second antibody for the analyte of interest. The result is that as the sample and labelled antibody is transported along the membrane or supporting phase, the analyte binds the antibody. This immune complex then at some point is transported in the liquid flow to the capture antibody which is immobilised at a given site within the filter or supporting phase. When the analyte-labelled antibody complex interacts with the immobilised capture antibody it is captured by binding to an available epitope on the analyte of interest, resulting in the immobilisation of the label which is attached to the labelled antibody and complexed with the analyte. The fluid continues to wick or flow past the immobilised antibody carrying any un-complexed (not bound to analyte of interest) labelled antibody away from the immobilised capture antibody. At this point, typically, the labelled antibody is detected. These assays use a number of different labelling methods such as particles and enzymes. In application of these immunochromatographic methods, as an advantageous embodiment of this invention, an ECL label could be bound to the antibody (IGEN, mc, Gaithersburg, MD) although other labels are possible, and the filter matrix or supporting phase could be manufactured to include structures of conductive and/or semi conductive materials. Examples of such conductive and/or semi conductive materials are carbon nanotubes, carbon fibres, glassy carbon particles, Au and/or Pt nanowires. These conductive materials would be mixed with the materials used in the manufacture of the filter membranes or fibre wicking material in such a way that they would be immobilised within the bulk of the membrane or supporting phase in such a way that the supporting phase or membrane is not transformed into an electronic conducting or semi-conducting material.

An alternative embodiment would be to immobilise the capture antibody onto a conductive or semi-conductive material (i.e. glassy carbon) and to impregnate the filter or supporting material with these particles at a given site. An example of this is described in US 5008080, but only uses plastic microparticles as a solid phase for antibodies impregnated into a glass fibre filter for use as a capture phase in an enzyme based immunoassay. These antibody-coated particles could, for example, be ink jet printed onto the filter or supporting material or pipetted. With a suitable selection of particles or fibres, nanotube sizes or aggregates, these would not migrate from the site of impregnation due to the relative size of the antibody-coated particles to the pores or channels in the filter or flow-supporting material. Alternatively, the conductive particles or fibres could be chemically or physically trapped in the filter or flow-supporting material by impregnation of the conductive or semi-conductive particles or fibres using printing or injection methods followed by antibody immobilisation either onto or within close proximity of the conductive or semi-conductive material. Potentially appropriate conductive and semi-conductive materials are carbon nanotubes, carbon fibres, C, Au and or Pt nanowires. With this combination of an ECL-labelled antibody and an electrochemically activatable filter or flow-supporting material, the assay would be carried out as described above for the traditional filter strip or wicking method (also called immunochromatographic tests or lateral-flow tests) but the detection of the antibody-captured analyte/antibody complex would be achieved via the application of a high voltage field (advantageously 2v/cm to 50 kv/cm, most advantageously 500v/cm to 50 kv/cm) across the filter or flow-supporting material such as to generate electrochemically active portions of the surface resulting in the generation of light via ECL. The ECL light is detected via a number of understood methods for light detection and or quantitation, including eye, photography, photodiode, photomultiplier or photocell.

It will be understood by those skilled in the art that this method of analyte detection using a binding reaction between antibody and analyte could also be applied without a labelled antibody but via the use of a labelled analyte which competes with the sample analyte for binding to the capture antibody. It is also understood by those skilled in the art that these immunoassay formats are applicable to numerous binding assays based on other well understood binding interactions, e.g. nucleic acid - nucleic acid, receptor-ligand, nucleic acid analogues-nucleic acid, and nucleic acid-protein. It is also understood that these immunochromatographic assays may be developed with liquid reagents not dried onto the immunochromatographic assay device.

In a further embodiment of the above method where the capture antibody or nucleic acid is attached to the conducting or semi-conducting material dispersed throughout at least a portion of the flow or filter material; the assay may be carried out without the need for a so-called wash step to remove the unbound or free label. In this case, the binding of the labelled species modulated in the presence of the analyte is detected due to the effective concentration of the label on the surface of the conducting or semi-conducting material which allows selective EC activation of the bound labelled species over that of the unbound. This method - which allows the assays to be carried out without a wash - is of great benefit in that it allows for rapid testing and simplification of the diagnostic system device.

In a further embodiment related to using a filter strip or wicking method (also called immunochromatographic tests or lateral-flow tests), the structures of conductive or semi-conductive material - advantageously with the longest dimension being less then 20µm - would be the label and a binding species attached to this label. In this embodiment, the assay would be conducted in the normal manner for such immunochromatographic tests or lateral-flow tests, resulting in the capture of the structures of conductive or semi-conductive material on the capture antibody at a predetermined site in the immunochromatographic tests or lateral-flow tests strip or cartridge based on the presence of analyte. Such assays already exist and make use of latex beads, colloidal gold or silver as a label on the antibody (Boehringer Mannheim) but these use visual detection of the particles and no use is made of the electrochemical properties of the conducting or semi-conducting material.

In this invention, we would carry out the immunoassay in a similar format but make use of, for example, a buffer containing ECL active species. When the binding reaction and capture to the immobilised binding species (antibody) is completed, the labels - which are conductive or semi-conductive materials - would be activated. The activation of the bound conductive or semi-conductive material would be achieved by the application of a high voltage (advantageously 2v/cm to 50 kv/cm, most advantageously 500v/cm to 30 kv/cm) across the binding zone in the immunochromatographic tests or lateral-flow tests. This application of a high voltage across the binding zone results in the formation of cathodic and anodic faces on the labels of conductive or semi-conductive materials used in the immunochromatographic tests or lateral-flow tests, and generates bipolar labels. The result of the formation of these bipolar labels is that they become electrochemically active and result in the activation of the ECL active species and the emission of light. This can be detected in numerous ways as described in this invention. It is also possible to generate other detectable signals using this format, e.g. fluorescence, chemiluminescence using luminol or acridinium esters which is a well understood method for the generation of light from an electrochemical reaction. Stabilised dioxetanes are also considered since they can be activated by electrochemical methods. Visible signals can also be generated e.g. by electropolymerisation to generate polypyrole and polythiophenes. The conductive or semi-conductive material used as the label may also be electroluminescent or chemiluminescent without addition of active species to the buffer used for the assay. An example of such an electroluminescent or chemiluminescent material is Al (Haapakka K et al, 1988, Anal Chim Acta 207, 195-210). It is also contemplated that other electroluminescent compounds may be used in this way. This mode of detection for the conducting or semi-conducting label is superior to known visual methods as the labels can be detected before they are visible or they can be amplified to become visible as in the case of electropolymerisation. Thus, the invention improves on the current immunochromatographic tests or lateral-flow tests which use conductive or semi-conductive materials as labels.

In an alternative embodiment, these filter wicking assays may also be applied to the detection of analytes without the need for a binding species. In these assays, ECL labels or co-reactants may be detected. Examples of potentially-detectable analytes have already be described above for the subject invention. A good example would be the detection of glucose, where the application of the sample to the filter-wicking substrate brings the glucose into contact with glucose oxidase and luminol. The glucose in the sample is acted upon by the glucose oxidase to generate hydrogen peroxide and this wicks up the wicking substrate and comes into contact with the structures of conducting and/or semi-conducting material of the subject invention. The wicking substrate with the sample is then subject to an applied external voltage which activates the structures of conducting and/or semi-conducting material to generate ECL from the luminol and the hydrogen peroxide produced by the action of glucose oxidase on the glucose in the sample. This ECL is used to detect and/or quantitate the glucose in the sample. The value of a glucose assay in this format is that it allows the separation of the sample prior to the enzyme reaction and detection of the ECL. This is most valuable as, for example in the case of a blood sample, it allows the removal of the red and white blood cells and other interfering substances. This glucose assay may also be carried out with dehydrogenases using the ECL from the Ru(bpy)₃²⁺ and NAD(P)H reaction using the coupled enzyme reaction employed for glucose analysis of hexokinase and glucose-6-phosphate dehydrogenase (as described above in analytes).

These filter wicking assays are thus not restricted to the detection of binding reactions but can be used to detect enzymes, substrates of enzymes, ECL co-reactants, ECL labels, ECL active molecules and EC reactive species as described earlier for the subject invention.

Assays making use of 1-10µm carbon structures (particles) in free solution.

An advantageous embodiment of the present invention is a binding assay such as an immunoassay, receptor, ligand or nucleic acid assay where small conducting or semi-conducting structures are coated with one of the binding species to form the capture phase of the assay. This coating may be made in numerous formats known in the art. Advantageously, glassy carbon (or graphitic) structures 1-50µm in diameter are activated by standard methods to generate, for example, NHS ester-activated structures (L. Dong et al 1996 J Mol, Rec. 9, 383-388). These NHS ester-activated structures are then allowed to react with the binding species of interest such as an antibody, receptor, ligand, antigen or nucleic acid. This results in the generation of a binding species coated onto the glassy carbon structure. Typically, any non specific binding in the assay is blocked by incubation with a non specific protein such as BSA and/or an amino-containing compound such as ethanolamine which react with any remaining NHS groups and bind to non-specific binding sites on the particle surface. These glassy carbon particles coated with a specific binding species such as an antibody (or streptavidin) and blocked are now ready to be used as the solid phase in a binding assay. Typically, these particles are added to a mixture of sample and another binding species which is labelled with an EC activatable label and able to bind to the analyte of interest. This mixture is allowed to incubate typically for 1-60 min. During this time, the labelled binding species, for example an antibody, binds to the analyte of interest and this complex is then captured by the binding species immobilised on the surface of the glassy carbon particles. This series of binding reactions results in the immobilisation of the EC activatable label on the surface of the glassy carbon particle which is then available to be activated. In one method for the activation, detection and/or quantitation of the bound label, the sample is now directly activated by the application of a voltage such that bipolar faces are generated on at least part of at least some of the glassy carbon particles. The bipolar faces so generated are of sufficient voltage that they are able to activate the EC activatable label(s). Typically, the EC activatable label is an ECL label which is detected using standard methods for the detection of light, e.g. PMT, photodiode, film, eye, CCD etc. In an alternative method of the above assay, the glassy carbon particles are first washed with a suitable buffer prior to the activation of the EC activatable labels as described above. The washing of the glassy carbon particles could be achieved in various ways. Using magnetic beads as the basis for the assay would allow for a method based on magnetic capture, but centrifugation and filtration are potentially valuable methods which could be applied. In the case of filtration, the beads could be readily collected and washed on the surface of a filter which could form the matrix on which the beads could be activated by the application of a voltage across the filter. The application of a voltage across a filter could make use of any axis and said axis of applied voltage may also advantageously be changed, rotated or scanned and the polarity of the field changed such that more of the captured structures and/or more of the structures coated with immobilised EC activatable labels are activated. For example, an alternating voltage may be applied with various frequencies and waveforms (or pulses). Additionally, the application of sonics may be used advantageously to improve signal generation, binding kinetics (i.e. antigen and antibody) and electrochemistry as has been demonstrated previously (Malins et al 1997, J Phys Chem, 101, 5063-8). These sonics may be from 1 to 100,000 Hz (cycles per sec), covering the range from sub-sonics to ultrasonics. Additionally, sonics can improve signals by inducing tumbling of the structures. This results in more of the surface being subjected to electrochemical activation. This assay format is ideal for all sandwich immunoassays and DNA probe sandwich assay formats. Also, the structures of conducting and/or semi conducting material may have a paramagnetic component allowing capture for washing and/or optimal positioning of the structures with respect to the voltage field and/or detector.

An alternative embodiment of the assay described above for glassy carbon beads is an assay that detects an analyte which is in competition with the bead-bound binding species for binding with a labelled equivalent of the analyte. The label in this example is the same as described above, being an EC activatable label which results in the production of a detectable signal or EC detectable species. In such an assay, the sample typically will have added to it the labelled equivalent of the analyte followed by the addition of the beads or other conducting or semi-conducting material coated with a binding species specific for the analyte of interest or capable of binding to a binding species specific for the analyte of interest. This mixture is then allowed to react typically for 1-90 min during which the analyte and the labelled analyte equivalent compete for binding to the immobilised binding species. During this competition step, the amount of bound labelled analyte equivalent is inversely proportional to the level of the analyte in the sample. Following this binding step, the bound electrochemically activatable and/or detectable label or moiety is detected as outlined in either of the two methods given above.

An example of such an assay for oestradiol is outlined below in the examples.

Scanning or alteration of the polarity and position of the bipolar faces may also be used to specifically generate ECL by rapidly generating oxidised and reduced species (i.e. Ru₃⁺ and Ru⁺). These are then able to react to yield the excited state which can then emit electromagnetic radiation. Also, the frequency of the applied voltage may be varied from 0 to 200,000 cycles per second, and advantageously from 0 to 5,000 cycles per second, to enhance certain reaction mechanisms, e.g. in the double potential step mode of ECL (Edge et al), and in luminol hydrogen peroxide electrochemically activated chemiluminescence.

It will be understood by those skilled in the art that numerous combinations of conducting and semi-conducting structures are possible and numerous assays are possible ranging from immunoassays, receptor ligand assays and nucleic acid probe assays to those used to detect substrates for NAD- and NADP-dependent enzymes, oxidases amines peroxides, oxalates and other ECL active molecules or co-reactants. In the case of the binding assay, the various formats give rise to the following compositions in the presence of analyte during the specific binding step(s);
1) structure-SA-Bio-Ab-At-Ab-El + Ab-El
2) structure-Ab-At-Ab-El + Ab-El
3) structure-At-Ab-El + At(in sample)-Ab-El
4) structure-Ab-At-El + structure-Ab-At + At + At-El
5) structure-SA-Bio-At-Ab-El + Ab-El + At(in sample)-Ab-El
6) structure-SA-Bio-Ab-At-El + structure-SA-Bio-Ab-At

Where:
Ab;
is selected from at least one of the following; antibody, nucleic acid, molecular species capable of binding in a sequence-dependent fashion to a nucleic acid sequence a so-called nucleic acid analogue or other nucleic analogue, or a receptor or ligand.
SA;
is selected from at least one of the following; streptavidin, avidin or a molecular species able to bind biotin or bind to Bio.
At;
is selected from at least one of the following; the analyte of interest which may be a ligand or nucleic acid.
El;
is the electrochemically active label.
Bio;
is biotin, amino biotin, imminobiotin peptide or a molecular species able to bind to SA.
Structure;
is selected from examples of structures of the subject invention described earlier.

An example of such assays is described below in the examples for TSH, HCG, oestradiol and nucleic acid assays.

### Multiple assays using arrays

Spatial arrays of multiple zones or elements are also contemplated by this invention. This consists of conductive and/or semiconductive structures of the subject invention spatially arranged such as on the surface of a non conductive surface such as glass or plastics. The methods for putting down or coating multiple elements or zones of conducting and/or semi conducting materials are well known in the art, e.g. sputtering, evaporation, electron beam evaporation, screen printing, painting, ink jet printing, other printing methods, photocopying, stamping, physically placing elements and fixing them in place, etc. It is also well known in the art that methods for coating, etching and recoating with conductive and/or semi conductive and non conductive materials are able to produce complex structures with exposed and masked regions which contain elements that are also connected electronically via a conductive and/or semi conductive material. It is also contemplated that spatial multi arrays of conducting and/or semi conducting materials of the subject invention could also be arranged in a 3 dimensional array, e.g. fabricated into a macroporous support or woven into a 3 dimensional array of supporting fibres.

The interest in generating such spatial arrays of structures conducting and/or semi conducting material of the subject invention is to allow for the construction of an analyte-detection device for multiple analytes where each of the structures of conducting and/or semi conducting material is a potential electrode capable of carrying out an assay for a predetermined analyte. In this way, the spatial arrays are able to carry out multiple analyte assays without the need for individual reaction chambers or tubes, or the need for multiple electronic conducting contacts to the structures of conducting and/or semi conducting material. In the array of structures of conducting and/or semi conducting material various materials can be mixed (see above) i.e. Pt, Au, Ag, Cu, C (and its various forms). Also, various methods for making the contemplated structures can be used in combination to achieve the optimal combinations of structures. The use of combinations of coating methods can also be used to develop combination materials such as platinized carbon by vacuum-deposition methods for Pt onto screen-printed Carbon structures. In addition, various sizes of structures of conducting and/or semi conducting material may be used to allow the sequential activation of various elements to provide an addition means for determining which assay is being read in addition to the spatial information from the structures' position within the spatial array. This use of various sizes of structures of conducting and/or semi conducting material - which will develop the potential required to activate the detectable electrochemistry of the subject invention - is achieved through the dependence of the structure size and the applied voltage in determining the voltage developed across a given structure within the applied voltage gradient. For example, a multi array device might be constructed with strips of carbon paste, (glassy carbon particles for example from 1-100µm in size, in a plastic or resin binder) or conductive composite. Alternatively, the methods used by Iwasaki may be used to generate thin patterned films of graphitic and conductive carbon (Y Iwasaki, Current Separations (1995) 14, 2-8 and O Niwa, Electroanalysis (1995) 7, 606-613). These strips of composite carbon could be various lengths such as 0.1, 0.2, 0.4, 0.8, 1.6 mm. These strips then placed into an instrument of the subject invention with the various lengths aligned such that the various long dimensions of the strips (0.1, 0.2, 0.4, 0.8, 1.6 mm) are lined up along an axis advantageously defined by the shortest distance between the externally and electronically connected electrodes. This array of potential bipolar electrodes when subjected to an increasing applied voltage in a solution of electrolyte within the central zone of an instrument of the subject invention would sequentially become electrochemically active. As the externally applied voltage is raised, the longest conducting structure or carbon composite will become active as it reaches the required voltage across the structures. This will be followed by the next length, and so on. When using a series of potential bipolar electrodes 0.1, 0.2, 0.4, 0.8, 1.6 mm long to induce an electrochemical reaction involving ECL with Ru (2, 2' bypyridyl)₃²⁺, the voltage applied by the externally electronically connected electrodes to the central zone of the instrument will double as each electrode becomes activated. In binding reactions, these various structures of conducting and/or semi-conducting materials of the subject invention would be coupled to binding species with the potential to present a different binding species on each element, thus enabling multiple analyte detection.

The detection and/or quantitation of multiple analytes on a single surface or with a single container is an advantageous embodiment of the subject invention. In these multiple assay applications, multiple zones or elements are fabricated onto a surface following conventional methods described for externally electronically connected interdigitated array microelectrodes (Y Iwasaki, Current Separations (1995) 14, 2-8 and O Niwa, Electroanalysis (1995) 7, 606-613) or constructed within a three dimensional structure such as a filter or fabric. Thus, it is envisaged that a sample would be introduced into a container, and within this container multiple zones or elements containing structures of conducting and/or semi conducting material of the subject invention not electronically connected to an external electronic circuit would allow multiple assays to be carried out. These assays would be based on the electrochemistry produced by the structures of conducting and/or semi conducting material of the subject invention not electronically connected to an external electronic circuit when subjected to a voltage gradient in a suitable electrolyte. Further applications of this embodiment are described below.

The multiple zones or elements for these multiple assays may consist of either a single structure of conducting and/or semi conducting material forming the zone, or multiple structures of conducting and/or semi conducting material grouped to form the zone or element in a defined region or portion of an assay component (surface or three dimensional array). In the case of the single structures, an example can be given where gold, carbon or other conducting or semi-conducting material is evaporated, sputtered, deposited, printed, painted or formed onto a surface making a defined shape based on a mask or printing device used in the process or by the use of resist patterning and selective etching. These defined shapes are electronically conductive and/or semi-conductive throughout the defined shape and form the zone or element in which an assay for an analyte may be conducted. In the alternative format, the multiple structures of conducting and/or semi-conducting material are used to from a zone or element for performing a given assay. Examples of this are the same as for the previous example but here, the mask or printing device, or the use of resist patterning and the selective etching used in the process, allows multiple structures to be deposited or formed in a given zone or element. Alternatively, the multiple structures of conducting and/or semi-conducting material may already be made and are simply applied to the surface such as with carbon fibres, fibrils, nanotubes or particles. These various methods result in the formation of multiple zones or elements (each able to carry out an assay) and each zone or element contains multiple structures of conducting and/or semi-conducting material.

In the case where multiple structures of conducting and/or semi-conducting materials are already formed and are then applied to create zones or elements containing multiple structures, these could be applied using various printing methods known in the art with or without binding material to bond the structures to the surface by physical immobilisation within a matrix (such as described in example 15 for the filter wicking assays). In the case where no binding material is used to physically trap the structures of conducting and/or semi-conducting material, these may be coupled to the surface either covalently or non-covalently. Using multiple structures of conducting and/or semi-conducting material within a zone or element to carry out an assay creates multiple bipolar electrode elements when subjected to an applied voltage field in the presence of electrolyte and provides for improved electrochemistry. In the case of physical immobilisation within a matrix, such as a filter, multiple analytes may be analysed in a flow-through or wicking format.

It is also contemplated that multi arrays may also be formed in a three dimensional array, e.g. using arrays of fibres incorporating structures of conducting and/or semi-conducting materials of the subject invention. In its simplest form, the structures of conducting and/or semi-conducting material could be woven into a fabric to form a filter or wicking material. The incorporation of structures into a fabric or filter is another example of physical immobilisation. These fibres may be made of fibre optical material allowing the lumination and/or collection of electromagnetic radiation from these conductive materials within a sample or within a matrix of such fibres. In this example, the structures of conducting and/or semi-conducting material may be advantageously coated onto the optical fibres.

For example, a surface may be patterned with gold and/or other conducting and/or semi-conducting materials as described above using various methods which are known, to form an array of single structures each of which acts as a potential bipolar electrode element for a given assay. In this case the gold forms a single structure of the subject invention which is also a single zone or region able to be become electrochemically activated by the application of a voltage field in the presence of a suitable electrolyte. Also, the surface may have zones which, rather than having a single structure of conducting material forming the zones or elements, may be composed of multiple structures within a defined zone or element for the means of conducting a specific assay.

By way of an illustration; in a practical example, a zone or element for carrying out a given assay might be defined as having a size of 5 mm by 5 mm; on a given surface these zones or elements may be spaced 2 mm apart. Thus, when constructing an assay device with 6 such zones, the surface would be at least 12 mm by 19 mm with the zones or elements in a 2 by 3 array. In this form, the 5 mm by 5 mm zones could be made of evaporated gold, etched carbon, glassy carbon particles or printed carbon nanotubes. The 5 mm by 5 mm zones may be a continuous electronically conducting structure but could also be composed of multiple structures of 5 µm by 5 µm contained within the 5 mm by 5 mm zones with spacing of 5 µm. This would create an array within the 5 mm by 5 mm zones of structures of conducting material. A 5 mm by 5 mm zone could contain 250,000 5 µm by 5 µm structures of conducting material spaced at 5 µm intervals.

The following example is to further illustrate the detection and/or quantitation of multiple analytes. In the case of glassy carbon particles, there is no need to define the structures of the conducting material on the surface as the particles already define the structures of the conducting material. Glassy carbon particles 1% (w/v) in polystyrene (10% w/v) dissolved in chloroform are applied to a polystyrene surface (15 mm by 20 mm by 2 mm thick) to generate the six 5 mm by 5 mm zones as described above. This surface, containing the 5 mm by 5 mm zones, is then etched using various methods to better expose the carbon and activate it for coupling to antibodies using various methods known in the art such as abrasion followed by chemical activation (see examples) or a plasma such as oxygen (O Niwa, Electroanalysis (1995) 7, 606-613). Antibodies are coupled to the oxidised carbon surface as described in the following examples. Specific antibodies are coupled to the 6 zones by spotting the specific antibody onto each zone. Following coupling of the antibodies to the zones, the polystyrene surface is blocked with a solution of bovine serum albumin and washed ready for use in an assay. The surface is then covered with the sample of interest and incubated for 1 hr followed by washing. The surface is then incubated with antibodies labelled with Ru(bpy)₃²⁺ for 1 hr followed by washing in PBS. The surface is then placed in a cell containing an electrolyte able to support ECL as described in the examples, and is subjected to a voltage field. The light emitted from each of the zones is detected using a CCD camera. The applied voltage field is advantageously applied in a thin film cell such that the surface of the polystyrene coated with the glassy carbon particles is parallel to the voltage field. The light generated at each of the zones is proportional to the concentration of the analytes of interest in the sample. In this way, multiple analytes are detected and may be quantitated.

### Nucleic acid assays

In an alternative embodiment, an assay for nucleic acids is contemplated. Here, a specific binding species for the nucleic acid is used, typically a nucleic acid sequence but this also could be a nucleic acid analogue.

Numerous examples of binding assay formats are known for various nucleic acid-based assays and all are amenable to the assay system of the subject invention. Some are outlined above in the schematic for binding assays. In its simplest form, the nucleic acid or DNA of interest (the sample containing the analyte of interest) can be coupled to the surface of the structures of conducting and/or semi-conducting material using methods known in the art via phosphate, amine, aldehyde or thiol groups on the nucleic acid. The DNA modified structures would then form the basis of the specific binding surface for the complementary binding nucleic acid or nucleic acid analogue labelled with the electrochemically detectable species, for example Ru(bpy)₃²⁺. The DNA modified structures are then subjected to a so-called pre-hybridisation reaction which effectively blocks the non-specific binding sites on the structure of conducting and/or semi-conducting material. Following this pre-hybridisation, the DNA modified structures are then subjected to the specific hybridisation reaction with complementary binding nucleic acid or nucleic acid analogue labelled with the electrochemically detectable species, for example Ru(bpy)₃²⁺. This hybridisation reaction between the structure-bound DNA and the complementary binding nucleic acid or nucleic acid analogue labelled with the electrochemically detectable species, for example Ru(bpy)₃²⁺, can last for 1 min to 24 hrs and depends on the concentration of the complementary binding nucleic acid or nucleic acid analogue labelled with the electrochemically detectable species, for example Ru(bpy)₃²⁺, the temperature and the ionic strength of the hybridisation buffer known in the art. After the hybridisation step, the structures are washed into an electrolyte able to support the detectable electrochemistry of the electrochemically detectable species. The structures are then introduced into the cell of an instrument of the subject invention and subjected to the voltage field applied to the central zone of the cell by the externally electronically connected electrodes. The detectable electrochemical reaction is then detected, for example by a PMT, CCD camera or photodiode in the case of a detectable electrochemical reaction based on ECL or chemiluminescence. The detection and/or quantitation of the detectable electrochemical reaction is used to determine the presence and or quantity of a nucleic acid sequence of interest in the analyte or sample nucleic acid. In the case of Ru(bpy)₃²⁺ and DNA, the ECL light level is used to determine the presence of a given DNA sample and or the number of genes present in the sample. It will be understood that the complementary binding nucleic acid or nucleic acid analogue labelled with the electrochemically detectable species, for example Ru(bpy)₃²⁺, may instead be labelled with a binding species such as biotin and then rendered detectable by binding of a binding species such as streptavidin coupled to an electrochemically detectable species, for example Ru(bpy)₃²⁺.

In an alternative format, known as sandwich hybridisation, a specific nucleic acid sequence (or nucleic acid analogue) for the analyte nucleic acid of interest is coupled to the surface of the structures of conducting and/or semi-conducting material using methods known in the art via phosphate, amine, aldehyde or thiol groups on the nucleic acid. The nucleic acid modified structures would then form the basis of the specific binding surface for the analyte nucleic acid of interest. The nucleic acid modified structures are then subjected to a so-called pre-hybridisation reaction which effectively blocks the non-specific binding sites on the structure of conducting and/or semi-conducting material. Following this step, the sample nucleic acid containing the analyte nucleic acid is added to these pre-hybridised structures either with, before or after the addition of a nucleic acid or nucleic acid analogue labelled with the electrochemically detectable species, for example Ru(bpy)₃²⁺, which is similarly complementary to the analyte of interest, and subjected to hybridisation.

The nucleic acid coupled to the structure and the nucleic acid labelled with the electrochemically detectable species are selected such that they hybridise to the analyte nucleic acid in such a way that the analyte nucleic acid in the sample links the structure-bound nucleic acid to the nucleic acid labelled with the electrochemically detectable species. For example, the nucleic acid coupled to the structure and the nucleic acid labelled with the electrochemically detectable species might be selected to bind to the same strand of the analyte nucleic acid without interfering with binding of each other to the analyte strand, typically within 1 kilobase of each other, and advantageously within 200 bases, most advantageously within 50 bases.

This hybridisation reaction of the structure-bound nucleic acid or nucleic acid analogue and the nucleic acid or nucleic acid analogue labelled with the electrochemically detectable species, for example Ru(bpy)₃²⁺, to the analyte nucleic acid in the sample can last for 1 min to 24 hrs and depends on the concentration of the nucleic acid or nucleic acid analogue labelled with the electrochemically detectable species, for example Ru(bpy)₃²⁺, the temperature and the ionic strength of the hybridisation buffer. After the hybridisation step, the structures are washed into an electrolyte able to support the detectable electrochemistry of the electrochemically detectable species. The structures are then subjected to a voltage field applied to the central zone of the cell by the externally electronically connected electrodes. The detectable electrochemical reaction, in the case of a detectable electrochemical reaction based on ECL or chemiluminescence, is then detected for example by a PMT, CCD camera or photodiode. The detection and/or quantitation of the detectable electrochemical reaction is used to determine the presence and or quantity of a nucleic acid sequence of interest in the analyte or sample nucleic acid. In the case of Ru(bpy)₃²⁺ and DNA, the ECL light level is used to determine the presence of a given DNA sample and/or the number of genes present in the sample.

In a further embodiment of the above example, the structures of conducting and or semi-conducting material are coated, coupled or derivatized with a binding species which binds to a binding species on a nucleic acid or nucleic acid analogue. For example, streptavidin could be coupled to the structure and the nucleic acid coupled to biotin. These two reagents replace the structures coated or coupled to a specific capture nucleic acid in the previous example and allow the hybridisation reaction to occur in solution concurrent with or followed by the capture of the hybrid complex of the three nucleic acids (the binding species-labelled analyte-specific nucleic acid, the analyte nucleic acid and the nucleic acid labelled with the electrochemically detectable species). This method also allows for the use of a universal structure and a specific capture oligonucleotide which provides for greater flexibility in the development of a random access analyser to assay a number of different analytes. This principle forms the basis of the Boehringer Mannheim Elecsys system where the structures (paramagnetic beads) are coated with streptavidin.

Other formats of nucleic acid detection which differ from the traditional binding reaction-based tests are those where the sample containing the analyte nucleic acid is subjected to an enzymatic step which allows amplification of the amount of the analyte nucleic acid in the sample and/or labelling of the analyte nucleic acid with either a binding species or an electrochemically detectable species. The following are some examples known in the art.

In the case of the polymerase chain reaction (PCR), one or more oligonucleotide sequences (primers) are used to prime the synthesis of nucleic acid resulting in the amplification of the analyte nucleic acid which is adjacent to the primers used in the PCR. The product of such a PCR is nucleic acid which can be detected and/or quantitated by the above methods. Alternatively, the primers used to amplify the analyte nucleic acid could be labelled with either a binding species or an electrochemically detectable species. In the case where the PCR includes a primer labelled with an electrochemically detectable species, the products of this reaction may be detected and/or quantitated by hybridisation using structures coupled to a nucleic acid sequence specific (and complementary) to the analyte nucleic acid sequence which is amplified by the primer labelled with the electrochemically detectable species. These structures coupled to a nucleic acid sequence are prepared as described above for the sandwich hybridisation. Also, it will be understood that these nucleic acid-coupled structures may be substituted with a combination of a structure coupled to a binding species (streptavidin) and a nucleic acid sequence specific (and complimentary) to the analyte nucleic acid sequence labelled with a complementary binding species (biotin) to the binding species coupled to the structure, as described above. It will be understood that the format just described could be modified such that the binding species is incorporated into the PCR and the electrochemically detectable species can be attached to the nucleic acid sequence specific (and complementary) to the analyte nucleic acid sequence which is amplified. It will also be understood that in addition to the PCR amplification described, the enzymatic synthesis of nucleic acid using primers may also be used to label nucleic acid analytes of interest with binding species and/or electrochemically detectable species using the primer. In addition, it is also well known that the labelling of nucleic acid with various species is possible via the use of direct incorporation of labelled NTPs and chemical modification of the nucleic acid (US5512433).

In an alternative format, the sample nucleic acid is hybridised to a nucleic acid or nucleic acid analogue which is able to form an antigen significantly different from either nucleic acid in the hybridised form such that an antibody will bind the hybrid and allow assay of the analyte nucleic acid. In this format, the analyte may be labelled with various species and/or the nucleic acid and/or the antibody specific for the analyte nucleic acid to probe the nucleic acid hybrid which is formed. An example of such a system is based on the formation of a DNA:RNA hybrid which is recognised by an antibody specific for the hybrid such as those described in US 4833084. It will be understood that various combinations of binding species and labels are possible. It will also be understood that by analogy, the use of triple helix binding may also achieve a substantially equivalent result by recognition of a specific hybrid.

In another nucleic acid assay format, the structures may be coupled to a nucleic acid sequence which is able to hybridise to an analyte nucleic acid either directly from a sample or after amplification using for example PCR, NASBA or other target-amplification systems. In addition to these nucleic acid sequences, a probe sequence labelled with an electrochemically detectable species complementary to the structure-coupled nucleic acid may be used. The analyte nucleic acid competes with the binding of the probe sequence labelled with an electrochemically detectable species for the structure-bound nucleic acid. A loss of structure-bound electrochemically detectable species results from hybridisation to the analyte nucleic acid. This results in a signal decrease. An obvious modification to this method would be to use a binding species on the structure (streptavidin) and a binding species on the capture nucleic acid (biotin) such that the nucleic acid is captured onto the structure at the desired time. The hybridisation steps, wash steps and analysis are as described in the examples above.

It is understood by those skilled in the art that the types of sample containing analyte nucleic acid of interest include; cells, biological fluids such as serum, plasma, urine, saliva, stool, semen, sputum, cell lysates, viruses, bacteria, plant tissue, animal tissues, fungi, achea bacteria, DNA, cDNA, RNA, mRNA, tRNA, rRNA, plasmids, phage, mitochondrial nucleic acid.

Examples of the above formats for nucleic acid assays based on conventional electrochemical activation using externally electronically connected electrodes have been described (Kenten et al 1991 Clin Chem 37, 1626; Kenten et al 1992 Clin Chem 38, 873; and Van Gemen et al 1994 J. Virol. Methods 49, 157).

### Flow-through analysis systems

In an alternative embodiment of the subject invention, a flow-through assay system is contemplated. This method is related to methods used for cell counting and fluorescent cell analysis but provides a novel solution to improving assay sensitivity and simplicity. In this assay system, an instrument is constructed which contains the basic elements described above containing a voltage control circuit designed to apply a voltage using the externally connectable electrodes which are contained in a cell, a detector for electromagnetic radiation which is a photomultiplier tube (PMT), a computer for the control of the voltage circuit, a detector and a pump. In addition to the elements described previously, the instrument contains a pump which controls - with the aid of the computer - the flow of the sample, and a cell which is designed to have a narrow zone (the central zone of the subject invention) through which will flow the structures of the subject invention which are advantageously conductive particles. The cell of this instrument in its basic form could be a simple tube with a neck or narrow portion such that the flow of sample containing the conductive particles results in the separation of the particles allowing individual particles to be present in this neck or narrow zone. This basic cell would have externally connected electrodes positioned either side of the central zone composed of the neck or narrow portion of the flow cell. This would place these electrodes up stream and down stream of the central measuring zone around the neck or narrow portion of the flow cell. Narrowing the flow path in this way creates a flow of single particles past the PMT which is positioned to view the narrow or neck portion of the flow cell. This narrowing of the flow cell to form a neck also creates an increase in the voltage gradient at this point such that the voltage across the conductive particles reaches a point at which a detectable electrochemical reaction can take place. Typically, this reaction generates light. The other advantage to a cell configured to form a gradual narrowing of the flow path is that the voltage gradient will increase across the conductive particles as they flow through this narrow neck-like region. This gradual increase in the voltage would allow the cell to activate particles of different sizes at different points as they flow through the narrowed portion of the cell. For example, the larger particles require a lower voltage gradient than do the small particles to achieve the required surface voltage to activate any given electrochemical process. Thus, as the larger particles travel in the flow of electrolyte through the narrowed portion of the cell they will be activated earlier than the smaller particles to carry out a given electrochemical reaction. This differential activation of particles within the flow cell under a given voltage applied from the externally connected electrodes also allows this instrument configuration to detect multiple analytes by determination of the position at which the particles become activated. For example, an assay would be constructed with particles of various sizes such that each binding species for each assay would be bound to a different sized particle. Each group of different sized particles would be activated at different positions in the flow cell as the sample flowed through under the voltage applied from the externally connected electrodes. This mixture of various sized particles coated with a number of different binding species would be mixed with a sample of interest and with labelled binding species to effect a binding of the labelled binding species proportional to the concentration of the analytes of interest in the sample of interest.

For example, each size of conducting particle would be coated with a different antibody. This would result in a mixture of particles of various sizes where a given particle size would have a given antibody coated on it. This mixture of coated conductive particles would then be added to a sample which contains a series of analytes to which the antibodies coated on the conductive particles could bind. In addition to the beads, labelled antibodies or labelled antigen (labelled with an electrochemiluminescent or chemiluminescent species) would be added to the sample either with the coated conducting particle, before or after. This mixture of coated conducting particles, sample and labelled antibodies and/or antigens would then be allowed to bind. This would result in the formation of antibody-antigen complexes on the surfaces of the various sized conducting particles and thus the immobilisation of the labels attached to the antibodies and/or antigens in amounts related to the amounts of the analytes in the sample. At this point, the sample may be analysed with or without addition of further buffers. Further buffers may be needed to assist in the optimal flow by dilution, and/or electrochemiluminescence and/or chemiluminescence of the coated conductive particles in the applied voltage field, This mixture would then be aspirated into the flow cell of the instrument as outlined above in this example. As it flows through the narrow portion of the flow cell, the sample will generate light from the various sized particles at specific positions in the flow cell path depending on the size of the particle. The location of the light is determined by the use of light detectors positioned such that light is detected at defined positions in the flow path. This detection of light can be achieved advantageously by the use of a diode array or similar as described earlier. In addition, the light emitted may be subjected to spectral analysis.

This flow-through assay system based on conducting particles thus offers great value as it provides multiple analyte detection and/or quantitation using particle size. This advantage of the flow-through system is enhanced by its ability to carry out multi-analyte detection using multiple wavelengths. Here, each particles light emission is analysed spectrally, and a spectral signature is all that is required to identify and thus assay a given particle. Since each particle is spectrally analysed as it passes the detectors, labels with overlapping spectra are readily detectable using, for example, in its simplest form, a 2 spectral window detection system. Such a system based on two spectral windows can readily detect multiple labels with minor differences in peak emissions when the labels are excited and analysed separately. Such a system has been used to detect the presence of at least 4 labels in a DNA sequencing system produced by DuPont. In the case of the system used by Luminex 64, different beads are detected based on two fluorescent dyes, one orange and one red.

An alternative cell configuration consists of electrodes which flank the narrow portion of the flow cell with a voltage applied across the path of the particle as it flows through the cell.

In an additional embodiment of the flow-through system described above, detection of the particle may also be incorporated into the instrument. A number of methods could be used based on methods currently employed for particle analysis or cell analysis. An example of this is the system used in the copalis technology (Sienna Biotech, Columbia MD) where size is determined by light scattering. Examples of such methods which could be advantageously incorporated in the flow-through system are given hereafter. Light or advantageously a laser could be used to detect and analyse the particles as they pass through the detection zone. This could be enhanced by the use of coloured or fluorescent structures of conducting and/or semi-conducting material of the subject invention to enable additional particle identification for multiple analyte detection in addition to the use of particle size and ECL wavelength. Such a system is used by Luminex (http://www.luminexcorp.com; Fulton et al Clin Chem, 1997, 43, 1749) where two fluorescent dyes are used to code beads into 64 different groups and a single green fluorescent dye is used for the various assays. The flow-through system of the subject invention could also make use of this method for particle identification but may also make use of an electrochemically detectable species, advantageously an ECL label. The addition of a light source to the flow-through instrument may not require a supplementary detector but could make use of the existing detector used for the ECL emission. In order to detect the ECL with the light from the light source, this light source could be pulsed at a given frequency such that the ECL and fluorescent light emission could be multiplexed and then discriminated by the light detector and analysis system. The advantage of this system is that it would provide information concerning the size and spectral properties of the structure of conducting and/or semi-conducting material of the subject invention and correlate this with the ECL for the particle. Thus, this system would allow for multiple analyte detection and/or quantitation. Based on results obtained with the Luminex system, 64 to 500 assays may be readily carried out with this type of system.

Alternatively, the particles may be detected and analysed as they flow through the neck or narrow zone by analysis of the resistance across the neck. This method has been widely used for particle and cell analysis invented by Coulter and is often called the aperture impedance or electrical method.

In an alternative embodiment of the subject invention, structures of the subject invention coupled to binding species may be used to bind to cells resulting in aggregation or the formation of multiple beads around a cell or multi-cell collection such as an embryo or micro-organism. These aggregates of more than one bead would then be detected based on the intensity of the light emitted as they pass through the elevated voltage zone which activates the electrochemistry on the surface of the structures. Advantageously, this analysis would make use of the structures as labels and most advantageously the detectable electrochemistry would be ECL. Thus, after binding the structures, the cells would be added to a buffer containing an ECL species and a coreactant if needed to allow ECL at the surface of the structures as they pass the activation and detection zone. Advantageously, the structures used in this embodiment would be beads, particles, nanotubes or fibrils. More advantageously, the structures would be nanotubes or fibrils. Most advantageously, the structures would be graphitic carbon nanotubes or fibrils.

In an alternative embodiment of the flow-through cell configuration of the subject invention, analytes may be detected for eluates from gels, HPLC, FPLC, chromatography and electrophoretic separation methods. In this embodiment, a flowing stream from the separation method would be mixed with a flowing stream of buffer, containing structures of the subject invention.

This mixture would then flow through a cell as described in the above embodiments. This would result in the activation of the structures of the subject invention to carry out electrochemistry.

The results of the activation of electrochemistry would generate a detectable signal. An example could be the detection of amines from HPLC as has been described (Forbes et al 1997, Analytica Chimica Acta 347, 289-293) using conventional electrochemical methods. For the detection of amines from an HPLC eluate, a flowing stream of Ru(bpy)₃²⁺ with the structures of the subject invention would be mixed with the HPLC eluate and then passed through a cell where the voltage gradient is applied as described above. Advantageously, the structures would be carbon fibres or nanotubes.

It will be understood that this mode of detection can be applied to any detectable electrochemical reaction by judicious choice of the known buffers and coreactants.

In general, the methods used to construct flow cells able to create a flow of individual particles through a zone or neck in the flow are well understood. Thus, the construction of variations which draw on the current knowledge for particle and cell analysers is understood.

### In situ binding assays

In a further embodiment of the subject invention, an alternative use of the structures of conducting and/or semi conducting materials as labels attached to a binding species is considered. In this example, advantageously, the structure is a carbon nanotube or fibril (advantageously 0.02 to 50 µm, most advantageously 0.5 to 5 µm in length) which is advantageously labelled with a binding species such as an antibody, nucleic acid, or receptor ligand. This binding species-attached carbon nanotube or fibril is then used as a reagent in binding to samples of binding species present on a surface. This may be the surface of a synthetic material but advantageously the surface is a biological material grown or deposited onto a surface such as a glass microscope slide. Examples of such are tissue sections, cell cultures, immobilised arrays of nucleic acid, smears of tissue which are processed to preserve the biological material of interest, stabilise the biological material and improve the presentation of the biological material. After specific binding of the binding species on the carbon nanotube or fibril to the biological sample, the surface is washed thoroughly and placed in the central zone of a cell which contains ECL reagents that may be in organic solvents. Advantageously, the ECL reagents are either tripropylamine and Ru(bpy)₃²⁺ or hydrogen peroxide and luminol or luminol, hydrogen peroxide and ferrocene. This cell, which contains the two externally connectable electrodes flanking the central zone, is place in an instrument as described earlier for the application of the external voltage and detection of the ECL light emission. In this case, the use of a microscope and CCD camera has certain advantages in the instrument in that it provides a spatial determination of the position of the bound carbon nanotubes or fibrils on the surface of the sample. This use of the subject invention allows for sensitive detection of binding species on a surface and also provide the potential to determine spatial information concerning the distribution of a given binding species on a cell or within a cell, or the counting of the given cell type within a population.

### Enzyme assays using structure-bound labels

The assays described are directed at the detection of binding reactions, or the detection of a soluble analyte directly or generated by a chemical or enzymatic reaction. It will also be understood that other chemical and enzymatic assays may also be carried out using a number of the formats outlined above. These chemical and enzymatic assays would be based on both synthesis reactions and degradation reactions.

For example, a synthesis reaction would be the action of DNA polymerase on a template nucleic acid initiated by a primer. In this example, the primer could be labelled with an electrochemically detectable species such as an ECL label and during the polymerase reaction a binding species could be incorporated such as biotin using a biotinylated NTP. The result of such a polymerase reaction would be a nucleic acid strand containing both a binding species and an electrochemically detectable species. This nucleic acid strand could then be captured onto a structure of the subject invention and analysed to determine the level of polymerase activity. Examples of synthetic enzymes are DNA, RNA polymerase, glyco-transferases, terminal transferases.

In the case of a degradation reaction, for example a protease or other hydrolase, the substrate could be coupled to the structure of the subject invention in such a way that it contains an electrochemically detectable species which is removable by the action of the specific protease or hydrolase. These modified structures are then incubated with the protease or specific hydrolase. Incubation in the presence of an enzyme able to hydrolyse the substrate, and thus remove the electrochemically detectable species, results in a loss of signal. Examples of hydrolases are glycosidases, nucleases, protease and lipases which may be used in assays as above.

The following non-limiting examples are given by way of illustrations and are not to be considered a limitation of this invention, many apparent variations of which are possible without departing from the spirit or scope thereof. It will be understood that either the enzyme or the substrate may be assayed using the methods outlined above.

### Examples

### Example 1

Example of ECL apparatus and a method for obtaining a detectable signal from electrolyte-suspended conducting particles.

Basically, the apparatus (illustrated in schematic no. 1 below) integrates a luminometer, a voltage control circuit and a cell with two externally electronically connected electrodes.

In schematic no. 1 above: 1 is a potentiostat (voltage generator), 2 is a platinum electrode, 3 is a buffer, 4 is the narrow cross section window of the cell, 5 is a luminometer, 6 is a photomultiplier or photodiode, 7 is a controlling computer.

Optionally, the apparatus may have a pump or other fluid-handling components and a flow-through cell with externally electronically connected electrodes.

The ECL apparatus consists of a cell with two electrodes at each end and a centre section which is visible to a photomultiplier tube (PMT) (Hamamatsu) powered by a Pacific Instruments Photometer. The data from this photometer is digitised and analysed by a computer (Dell Corporation XPS 200, software: National Instruments, Austin Tx). The voltage applied to the two external electrodes (from a Spellman SL series high voltage generator) is controlled by the computer in such a way that the voltage applied to the centre section of the cell during a measurement cycle is correlated with the light level detected at the PMT. A voltage gradient of up to 35 kv/cm is applied across the central section of the cell which contains the sample to be measured. For safety reasons, the voltage control is constructed to prevent arcing with high current. The centre section of the cell, visible to the PMT, is designed with a narrow cross section such that most of the voltage gradient applied across the two externally electronically connected electrodes is present in this section. The sections of the cell which contain the externally electronically connected electrodes are designed such that they are not visible to the PMT during a measurement. This instrument is designed for multiple cell configurations to be used:

### Cell 1

One cell has a continuous channel from one end to the other which allows samples to be introduced using a pump or other flow control method. In this case, the two externally electronically connected electrodes are attached to the cell via a porous frit, permeable or semi-permeable diaphragms or membranes such that electrical contact is made through an electrolyte to the centre section which forms the measuring zone. This cell configuration allows samples to be placed in the measuring zone and then excited via the applied electric field between the two externally electronically connected electrodes. The light emitted is detected and the sample then flushed from the system followed by cleaning and introduction of the next sample for analysis. This cell configuration has windows in the measuring zone which would also permit fluorescence and spectrophotometric measurements to be made.

### Cell 2

The second cell is configured as in the first cell with respect to the externally electronically connected electrodes but does not have a continuous channel from one side to the other. Instead, the sample is introduced via a port in the centre section of the cell. The externally electronically connected electrodes are isolated from this centre section via a porous frit or semi-permeable membrane. This cell allow manual washing and introduction of the sample followed by a measurement cycle.

### Cell 3

The third cell is designed as for the second cell but the centre section is modified such that it is able to accept or hold a filter paper or immunochromatographic filter element or gel. This centre section is able to accept the filter paper or immunochromatographic filter element or gel in such a way that electrolyte is in contact with the filter paper or immunochromatographic filter element or gel and the externally electronically connected electrodes. This centre section with the filter element also has a window to hold the filter element in place and to allow close proximity of the PMT to the filter element, improving photon capture by the PMT. The window element is variously glass, plastic, and plastic wrap which is transparent to the photon wavelengths of interest.

The above instrument is also configured to allow the use of a photodiode in place of the PMT.

To test the above ECL apparatus and cells (cell 2), a suspension of glassy carbon spheres 20-50 µm (#41260, Alfa Aesar) at 20-200µg/ml in ECL buffer (200 mM potassium phosphate, 100 mM tripropylamine, 0.02% (w/v) Tween 20, pH 7.2) with 1nM Ru (2,2-bipyridyl)₃²⁺ [Ru (bpy)₃²⁺] is introduced manually into cell 2 through the port in the centre section of the cell. The externally electronically connected electrode chambers are filled with ECL buffer. The cell is then placed in proximity to the PMT such that it can substantially see the centre section of the cell but not the chambers which contain the externally electronically connected electrodes. The PMT and cell combination is placed in a dark environment to allow the specific detection of the light emitted from the ECL at the surface of the glassy carbon spheres. The results of this experiment demonstrate a peak of detectable light which is dependent on the level of the applied voltage. In this case, light is seen when a voltage of up to 3 kV/cm is applied in the form of a ramp of increasing voltage from 0 to 3 kV/cm. The ECL signal is found to increase with increasing numbers of particles and with increasing concentrations of Ru(bpy)₃²⁺.

### Example 2

### Chemical activation of fibrils, fibres, particles and other structures

Carbon materials (particles, fibres, nanotubes, fibrils) are obtained from Alfa Aesar (Ward Hill, MA) and MER corporation. These carbon materials are made into an aqueous slurry by mixing 0.1% w/w with deionized water and dispersing by sonication using a 400 watt sonication probe for 0.5 to 1 hour. These carbon materials are then ready for chemical activation by oxidation using chromic acid, perchioric acid (Dong et al 1996, J Mol Rec. 9, 383-388) (to introduce carboxyl groups) or for direct coating with biomolecules. Carbon fibres and various carbon particles and other such structures are obtained from Alfa Aesar (Ward Hill, MA). This oxidation is achieved following standard protocols as follows. For example, 10 g of the carbon fibrils, particles, fibres or other structures are mixed with 450 ml of conc. H₂SO₄. This preparation is then mixed with 8.68 g of NaClO₃ under argon by adding small amounts over a 24 hr period. The mixture is then added to ice and followed by extensive washing. The final carboxylated structures are dried and a portion used to determine the carboxylic acid content by titration of these groups. The chromic acid protocol is similar except that the reaction is not conducted under argon.

### Example 3

### Immobilisation of proteins on carbon particles, fibres and fibrils and other structures using non covalent methods

Carbon material suspensions as described in example 1 are added to PBS to generate a 0.1-1 mg/ml suspension of carbon. These are washed once then antibody or streptavidin is added at a concentration of 100-300µg/ml in a pH 9.6 100 mM sodium carbonate/bicarbonate buffer. The fibril mix is left to mix at 48°C for 16-20 hours. Following this incubation, the fibrils are washed by centrifugation and resuspended in 3% BSA, 0.1% Tween 20 PBS and incubated for 2 hours to block any uncoated sites. These fibrils are then washed 5 times in the 3% BSA, PBS with 0.05% sodium azide and finally resuspended in this buffer for storage. Prior to use, the fibrils are washed into PBS 0.1% Tween 20 to a concentration of 0.1-1 mg/ml.

In the case of other carbon particles, these are prepared by a quick wash in PBS followed by addition of the antibody-containing solution and application of the protocol above.

### Example 4

### Immobilisation of proteins on carbon (structures) particles, fibres and fibrils using covalent methods

Carbon particles, fibres and fibrils are obtained from MER corporation and Alfa Aesar (Ward Hill, MA). These are activated using oxidative methods such as perchloric acid, chromic acid treatment, oxygen plasma or other methods known in the art to generate -COOH functional groups on the surface of carbon, glassy carbon and graphitic carbon (see example 1). These carbon surfaces - derivatized with COOH groups - are suspended in anhydrous dioxane with mixing to concentrations of between 0.1 and 10 mg/ml. An estimate of the moles of COOH groups per mg of particles is made by titrating the COOH groups on a sample with NaOH. To this mixture is added a 5-30 fold molar excess of N-hydroxysuccinimide followed by mixing to obtain dissolution. When the N-hydroxysuccinimide has dissolved, an equivalent molar amount of ethyl-diamino-propyl-carbodiimide (EDAC) is added and the mixture incubated with mixing for 1-4 hours at room temperature (20-24°C). Following this incubation, the particles are separated from the mixture either by filtration (particles larger than 20µm) or by centrifugation. The particles are washed with anhydrous dioxane several times followed by an anhydrous methanol wash. These resultant NHS ester-activated particles are then dried under vacuum and stored under anhydrous conditions until used for coupling

For coupling, between 10 and 1 mg of dried NHS-ester particles are washed with PBS and added to a solution of the biomolecules to be coupled, typically streptavidin, antibody and amino-modified DNA or oligonucleotide. In the case of streptavidin, a 5-10 mg/ml solution in PBS is used. For antibody, between 0.5 and 1 mg/ml of antibody is used to couple to 1-3 mg of particles. For amino-modified oligonucleotides, between 0.1 and 1 mg/ml of oligonucleotide between 15 and 40 bases long is used. The particles and biomolecules are mixed to form a suspension and this is then incubated for 1-2 hours at room temperature. The coupling reaction between the amino groups on the biomolecules and the NHS-ester groups on the particles forms an amide bond between the biomolecule and the particle. These particles are then resuspended in 3% BSA, 0.1% Tween 20, PBS and incubated for 2 hrs. They are then washed and resuspended in the same buffer.

### Example 5

### Labelling of proteins with ECL labels

1 mg of protein is labelled with Ru (2,2-bipyridyl))₃²⁺ (Ru(bpy)₃²⁺). The protein is buffer-exchanged using Centricon 30 microconcentrators (Amicon) into 0.15M potassium phosphate buffer, 0.15M NaCl pH 7.8, the final volume being 0.5 mL. Immediately prior to use, 0.5 mg of Ru(bpy)₃²⁺-NHS (Ru(2,2-bipyridyl)2(4-[3-(1,3-dioxolan-2-yl)propyl]-4-methyl-2,2-bipyridine)²⁺) is dissolved with 125µl of anhydrous dimethyl sulfoxide (Aldrich). A molar ratio of about 25:1 should be achieved between the Ru(bpy)₃²⁺ and the protein, based on molecular weights of 1057 for Ru(bpy)₃²⁺-NHS and 150,000 for antibodies. The Ru(bpy)₃²⁺-NHS (45µl) is added to the protein solution while shaking. The ranges for labelling can be from 3:1 to 30:1 molar ratios depending on the protein being labelled. The reaction tube is incubated in the dark at room temperature for 30 minutes while shaking. The reaction is terminated by the addition of 25µl of 1M glycine and incubation for 10 minutes. The reaction mixture is purified by passage through a Sephadex G-25 column (1 X 20 cm in 0.15M potassium phosphate, 0.15M NaCl with 0.05% sodium azide pH 7.2). The Ru(bpy)₃²⁺-labelled protein fractions are collected and pooled. These labelled proteins are then diluted into PBS buffer with azide to give protein concentrations between 1.6µg/ml and 32µg/ml.

### Example 6

### HCG assay

Immunoassay for the quantitative determination of human chorionic gonadotropin. This electrochemiluminescent assay demonstrates the principles of the subject invention.

The measurement of HCG concentrations can be used to diagnose pregnancy just one week after conception.

Reagents are obtained from Boehringer Mannheim, i.e. HCG STAT immunoassay reagents (Cat #1731 289) sufficient for 100 tests. This test kit is used to provide the two antibodies, one being labelled with an electrochemically detectable species (Ru(bpy)₃²⁺), the other being a bifunctional binding species composed of an antibody conjugated to biotin. The solid phase corresponds to the glassy carbon beads prepared in examples 3 and 4 and coated with streptavidin. In addition, the HCG STAT calibrator set (Cat # 1731670), the buffer to provide optimal ECL, i.e. ProCell (Cat # 1662988) (this buffer is equivalent to the ECL buffer used in example 1 and to ORIGEN assay buffer) and Elecsys Diluent Universal (Cat # 1732277) are obtained from Boehringer Mannheim.

The samples are prepared from the HCG STAT calibrator set by dilution in Elecsys Diluent Universal. One of the calibrators in the HCG STAT calibrator kit consists of about 10 mIU/ml and the other about 5000 mIU/ml. The highest calibrator is used to make a series of samples by serial dilution in the Elecsys Diluent Universal to give a sequence of HCG values which form the basis of the demonstration. The result of the dilutions is a series of HCG values: 5000 (from the original calibrator), 1250, 625, 156, 78 and 20 mIU/ml.

The assay is performed as follows; 40 µl of the diluted calibrator described above is added to 220 µl of the biotinylated antibody and 220 µl of the Ru(bpy)₃²⁺-labelled antibody. This is mixed and incubated for 5-10 min followed by addition of 100µg (100 µl) of streptavidin-coated 2-4µm glassy carbon particles as prepared in examples 3 and 4. This mixture is incubated for 5-10 min with mixing. This mixture of assay components is then centrifuged and resuspended in the ProCell buffer and washed again before final resuspension in 600 µl of the ProCell buffer. These samples in the ProCell buffer are then introduced into the cell of the instrument as described in example 1 and the light given off for each sample is determined. The values are background-subtracted using the 0 HCG level sample and normalised to a value of 1000 for the 5000 mIU/ml calibrator sample. The results demonstrate an increase in light detected with increasing HCG levels in the samples.

### Example 7

### 17-beta Oestradiol (oestradiol) assay

Immunoassay for the quantitative determination of oestradiol. This electrochemiluminescent assay demonstrates the principles of the subject invention in a competitive assay format.

Measuring oestradiol concentrations is important in the determination of fertility disorders derived from the hypothalamus-pituitary-gonad axis, gynecomastia and tumours.

Reagents are obtained from Boehringer Mannheim Oestradiol immunoassay reagents (Cat #1776002) which contains reagents for 100 tests. This test kit is used to provide the two binding species for the assay: oestradiol with a peptide linker labelled with an electrochemically-detectable species (Ru(bpy)₃²⁺), an antibody used as a bifunctional binding species and another antibody conjugated to biotin. The solid phase corresponds to the glassy carbon beads prepared in examples 3 and 4 and coated with streptavidin. The Oestradiol CalSet (Cat # 1776037), the buffer to provide optimal ECL, ProCell (Cat # 1662988) and Elecsys Diluent Universal (Cat # 1732277) are obtained from Boehringer Mannheim.

The test samples are prepared from Sigma reference standard oestradiol (Cat # E 1132) made up in Elecsys Diluent Universal. In addition, test samples from the Oestradiol CalSet (Cat # 1776037) are run in some experiments.

The assay is performed as follows; 100 µl of the sample are added to 180 µl of the biotinylated polyclonal anti-oestradiol antibody from the kit and incubated for 10-15 min followed by addition of 180 µl of the oestradiol-peptide linker-(Ru(bpy)₃²⁺) and 100 µl of streptavidin-coated glassy carbon particles (100µg of 2-4µm coated glassy carbon particles prepared as in examples 3 and 4). This mixture is incubated for 10-15 min with mixing. This mixture of assay components is then centrifuged and resuspended in the ProCell buffer and washed again before final resuspension in 600 µl of the ProCell buffer. These samples in the ProCell buffer are then introduced into the cell of the instrument as described in example 1 and the light given off for each sample is determined. The values are background-subtracted using a sample not containing any oestradiol. The results demonstrate an increase in light detected with increasing oestradiol levels in the samples.

### Example 8

### TSH assay

100µl serum calibrators for TSH, 25µl of ECL-labelled mouse anti-TSH prepared as in example 5 (typically the amount of antibody added to a test is between 40 ng and 800 ng) and 25µl (between 3 and 100µg) of glassy carbon structures (1-10µm) coated with sheep anti-TSH in ECL buffer following the protocols given in examples 3 and 4, are combined and incubated in polypropylene tubes for 15 minutes at room temperature with mixing. The glassy carbon structures are then washed twice by centrifugation and resuspended in ECL buffer to a final volume of 1 ml. These samples are then analysed for ECL using the instrument as described in example 1. The ECL signals from the various samples are then plotted against the serum calibrator values to generate a standard curve. This demonstrates that the method allows the determination of analytes using simple binding interactions as seen in antibody-antigen, receptor-ligand and nucleic acid-nucleic acid binding reactions. This standard curve can then be used to determine the level of unknown samples of the TSH analyte.

This assay was repeated without washing the glassy carbon structures and demonstrated the use of this format for a no-wash, one-step assay without particle separations and/or washings.

### Example 9

### DNA probe assays

Oligonucleotides are obtained from Oligo etc (Wilsonville, OR, USA). Amino-modified oligonucleotides are labelled with Ru (2,2-bipyridyl)₃²⁺ using the NHS ester or made directly during synthesis using Ru (2,2-bipyridyl)₃²⁺ phosphoramidite obtained from IGEN Inc.

Beta-actin PCR, nucleic acid probe assay.

The PCR detection of beta-actin mRNA is achieved by the conversion of mRNA into cDNA followed by a PCR which includes a biotinylated primer and an ECL labelled primer. In this protocol, the ECL label used is ORIGEN TAG (IGEN, Gaithersburg MD). The following primers are used to amplify the beta-actin cDNA by PCR; 5' Biotin-GCC ACA GGA TTC CAT ACC CAA-3' and 5'ORIGEN TAG-GAG AAG AGC TAT GAG CTG CCT GAC-3'.

From 0.1 to 5 micrograms of mouse liver total RNA, including controls not containing any beta-actin, are reverse-transcribed in a 20µl reaction for 1 hr at 42°C with Superscript II RNase H- reverse transcriptase, oligo dT, and 10mM dNTP mix as recommended by the manufacturer (Life Technologies) to generate the cDNA. One microliter of cDNA is amplified in a 100 µl reaction containing 1XPCR buffer (Perkin Elmer), 200 µM dNTPs (LTI), 20 pmoles of each primer, and 4 units of Amplitaq DNA polymerase (PE) pre-mixed 1:1 with TaqStart Antibody (Clontech). Amplification is performed on a Perkin Elmer Model 480 Thermal Cycler using the following conditions: 10 minutes at 50°C; 10 minutes at 94°C; 20 cycles of 45 seconds at 94°C, 45 seconds at 64°C, 2 minutes at 72°C followed by a final extension of 7 minutes at 72°C. Following the PCR, 3 µl of the reaction mixture are added to 2-40 µg of streptavidin-coated glassy carbon structures (as prepared in examples 3 and 4) in 50µl of ECL buffer. This mix of PCR products and the streptavidin-coated glassy carbon structures is mixed for 30 minutes at room temperature followed by the addition of 250 µl of ECL buffer. Following this dilution step, the glassy carbon structures are washed in ECL buffer and then placed into the central section of the ECL instrument described in example 1 for ECL excitation and detection. The results demonstrate an ECL signal dependent on the level of the beta-actin DNA in the samples.

### Example 10

### Cholesterol assay

Solutions containing cholesterol (30µl of 2-50µM), cholesterol oxidase (10µl of 48 units/ml) and buffer (120µl of 100 mM NaPhosphate, pH5 containing 0.05% Triton X-100) are prepared and incubated for 30 min. The mixture is then rendered 0.2 mM with respect to luminol using a solution of luminol in 0.05M sodium borate buffer pH 10.0. In an alternative assay format, following the 30 min incubation step, the mixture is rendered 100µM ferrocene, 100µM luminol and 0.1 M Tris buffer. To these mixtures are added 2-80µg of glassy carbon particles (Alfa). The samples are then placed into the ECL instrument described in example 1 followed by excitation and detection of the ECL light. The samples show an increasing signal with increasing cholesterol, forming the basis of a cholesterol assay. Stock solutions of cholesterol are prepared by dissolving cholesterol in a mixture of Triton X100 and ethanol (3:2 v/v). The particles used in this assay are untreated 0.2-5µm glass carbon beads.

### Example 11

### Glucose assay

The basic reagent mix for the glucose assay contains 16.8 µg/ml hexokinase, 20 µg/ml glucose-6-phosphate dehydrogenase, 1 mM ATP, 1 mM NAD⁺, 2 mM MgSO₄ and 25 mM Tris HCL, pH 7.5. Aliquots of this reagent mix are prepared (270µl) and 30µl of various glucose standards are added to give a final concentration range of 5 to 35µM. These samples are incubated for 10 min at room temperature. Following this incubation, 30 µl of 10mM Ru(bpy)₃²⁺ is added followed by 2-40µg of glassy carbon particles (Alfa). The samples are then placed into the ECL instrument described in example 1 followed by excitation and detection of the ECL light. The samples show an increasing signal with increasing glucose, forming the basis of a glucose assay. The particles used in this assay are untreated 0.4-12µm glass carbon beads.

### Example 12

### Betalactamase assay

A solution of benzylpenicillin (1 mM) is prepared in 0.1 M phosphate (sodium salt), 0.05% Triton X-100 pH 7.5. This solution is rendered 10µm with respect to ruthenium (II) tris (bipyridyl) (Ru(bpy)₃²⁺). This mixture is used as the buffer for the detection of betalactamases. To 1 ml aliquots of this mixture are added E. coli beta lactamase RTEM to a concentration of between 1 and 10 nM. The enzyme reaction is allowed to proceed for about 10 min at room temperature (about 20°C). After the enzyme incubation, 2-40µg of glassy carbon particles (Alfa) are added and the mixture is placed in the central zone of the cell of the instrument described in example 1. The sample is then subjected to an applied voltage and the ECL signal is measured. It is found that ECL signal is significantly higher in the presence of the beta-lactamase enzyme. This allows a method to be developed for the detection of beta-lactams and betalactamases. The particles used in this assay are untreated 0.4-12µm glass carbon beads.

### Example 13

### Preparation of Antibody-Coated Carbon Structures

Carboxylate-modified carbon structures prepared as in example 2, (2.5µg) are added to 1.0 millilitre (ml) of methyl ethyl sulfonate (MES) buffer (5 millimolar (mM), pH 4.75) and 75 microliters of antibody solution (antibody to beta-hCG, Biogenesis UK) (2 milligrams per millilitre (mg/ml)). The solution is stirred and 100 ml of 1-Ethyl 3(3-Dimethyl-aminopropyl) carbodimide HCl (EDAC) (2 mg per 10 ml H₂O) are added. The solution is stirred overnight at 2-8°C, after which the structures are isolated by centrifugation, washed twice with 0.1% "Tween-20" solution, and resuspended in "PBS" Phosphate Buffered Saline (0.01 M KH₂PO₄; 0.15 M NaCl: pH 7.2) to yield a 0.125% solution. After resuspension in PBS, the structures are stored at 2-8°C for subsequent use in the following procedures.

### Example 14

### Preparation of Solid-Phase Reaction Matrix

50 microliters of the antibody-coated structures from Example 13 are added dropwise to the centre of a Whatman GF/D glass filter; 100 microliters of pig sera are then added and the filter and microparticles incubated for 30 minutes in a humidity chamber at room temperature. After this time, the filter - now containing the microparticles - is washed three times in 300 microliters of PBS buffer. The filter is then stored in a humidity chamber for use in the following immunoassay example. The microparticles are observed, by scanning electron microscopy, to have been trapped or agglomerated on the glass fibres of the filter material.

It should be noted that, in addition to the techniques described in the foregoing example, antibody, antigen, ligand, receptor and nucleic acid binding sequences may be attached to the particles by a variety of methods, e.g., adsorption or use of various chemical activators. Also, it is to be appreciated that the particles can be added to the fibrous matrix after, for example, animal sera or other blocking agents have been added, and that the use of such sera is not of critical importance. Therefore, the order of addition of the particles to the matrix and treatment thereof after or before incorporation into the matrix is not critical to the present invention. Moreover, it will be appreciated that fibrous materials can be used in place of the glass filter matrix material specifically described herein, and the advantages of the invention can also be realised thereby.

### Example 15

### Immunochromatographic Immunoassay Protocol (Determination of beta-hCG)

The glass fibre material containing the antibody-coated structures as previously described (example 14), is cut into substantially circular "disks", and the disks - forming reaction matrices - are placed in contact with a blotter material to absorb excess fluid from solutions used in the assay. Thereafter, five drops of test samples of human urine (about 280 microliters) containing zero, and 50 and 100 mIU/ml levels of beta-hCG (Table 1, infra) are added to each matrix after passage of the sample drops through a prefilter situated above each matrix. Three drops of an antibody labelled with an ECL active species, as used in example 6, are then added to each matrix through the prefilter, and each matrix is incubated at room temperature for about two minutes. The prefilter is then removed and 1.0 ml of PBS, 0.1% Tween 20 wash solution is added to each matrix to remove any excess antibody-enzyme conjugate. One drop of ProCell buffer is then added to each matrix. After two minutes, each matrix is placed in a cell (example 1, cell 3) containing two externally and electronically connected electrodes (see above description of ECL instruments). The matrix placed in the cell forms an electrical contact between the externally connected electrodes via the ProCell buffer which forms the electrolyte.

Each matrix is then subjected to an externally applied voltage. This voltage is applied at such a voltage gradient up to 10 kv/cm that at least some of the structures trapped in the glass fibre filter are rendered bipolar. These bipolar structures activate the ECL from the captured antibody labelled with an ECL active species and light is detected for the test samples which contains beta-hCG. The amount of light is correlated to the level of beta-HCG in the sample.

### Example 16

### Chromatographic assay with particles as labels

Glassy carbon particles are purified using sucrose gradient centrifugation to isolate particles within a range of 0.2 to 2µm in diameter. These particles are then activated using the oxidation reactions described above and activated to form the NHS ester as described. These beads are then reacted with BSA biotin from Pierce (Rockford, IL, cat # 29130). The resultant particles are then reacted with excess BSA to block any remaining binding sites and the beads are washed 5 times over a period of 3 days. Large pore nitrocellulose FF85 from Schleicher and Schuell (Ecquevilly, France) is cut into strips 1 cm by 10 cm and a strip of streptavidin applied 3 cm from one end of the nitrocellulose membrane. These membranes are then washed in water and incubated in 3% BSA in PBS overnight followed by washing in water and air-dried. These membranes are then placed into tubes containing 0.3 ml of the above biotinylated glassy carbon particles with and without added biotin and allowed to stand for 2 hrs such that the glassy carbon particles may wick up the dry membrane. The membranes are then washed with a solution of 0.1 mM luminol, 1 mM hydrogen peroxide, 0.1 mM ferrocene monocarboxylic acid (Sigma) and 0.05M Tris buffer pH 8. The membranes are then trimmed to remove the lower and upper portions of the strip to leave the site of streptavidin strip. The streptavidin strip portion is placed into a cell designed to provide electrolyte contact to two externally electronically connected electrodes attached to a voltage control circuit contained within the instrument as described in example 1. These membranes are then subjected to the voltage field and the ECL is detected. The strips run in the absence of added biotin show higher ECL signals than those run in the presence of free biotin which blocks the binding of the biotinylated glassy carbon particles and prevents the binding of these to the immobilised streptavidin on the nitrocellulose membrane. The binding of the glassy carbon particles immobilises a conductive structure of the subject invention which is then able to activate ECL when an external voltage is applied. This therefore demonstrates the potential of this system to form the basis of a system used to carry out specific binding reactions with enhanced sensitivity.

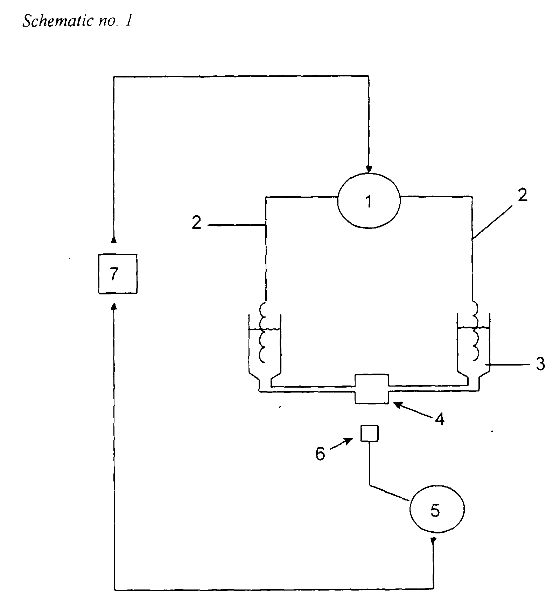

## Claims

1. An assay method for an analyte of interest using an electrochemical process, the assay comprising:
a) the establishment of contact between the sample to be assayed for an analyte of interest and at least one structure of conducting and/or semi-conducting material not in electronic contact with an external electronic circuit,
b) the application of a voltage gradient across at least a portion of said at least one structure of conducting and/or semi-conducting material not in electronic contact with an external electronic circuit, said voltage gradient being such that at least one electrochemical reaction takes place on at least one of said at least one structure of conducting and/or semi-conducting material not in electronic contact with an external electronic circuit,
c) the detection and or quantitation of said at least one electrochemical reaction directly and/or indirectly,
d) the determination of the presence and/or quantity of said analyte of interest.

2. An assay method as in claim 1 wherein said at least one structure of conducting and/or semi-conducting material is coupled to a binding species.

3. An assay method as in claim 1 wherein said electrochemical reaction results in electrochemiluminescence and/or chemiluminescence.

4. An assay method as in claim 2 wherein a binding species free in solution and coupled to at least one electrochemically detectable species is also added to said sample either together or before or after said at least one structure of conducting and/or semi-conducting material.

5. An assay as in claim 4 wherein said at least one electrochemically detectable species is able to support electrochemiluminescence or chemiluminescence either directly or indirectly.

6. An assay as in claim 5 wherein both said binding species are selected from the following; antibody, antigen, nucleic acid, receptor, ligand, lectin, nucleic acid analogue, streptavidin, avidin.

7. An assay as in claim 6 wherein said at least one structure of conducting and/or semi conducting material contains one of the following; carbon, gold, platinum.

8. An assay as in claim 7 wherein said at least one structure of conducting and/or semi conducting material is made from carbon in the form of graphitic carbon fibres, fibrils or nanotubes, graphitic particles, glassy carbon particles, carbon films, sputtered carbon, carbon pastes, carbon composites.

9. An assay as in claim 8 wherein said at least one structure of conducting and/or semi conducting material is a glassy carbon particle.

10. An assay as in claim 1 for substrates of enzymes or enzymes wherein said sample is mixed with at least one enzyme for at least one substrate and/or with at least one substrate for at least one enzyme either prior to or during the contact to said at least one structure of conducting and/or semi conducting material such that the products of said at least one enzyme action on said at least one substrate are electrochemically detectable species.

11. An assay as in claim 10 wherein said at least one substrate for at least one enzyme is a substrate for either a dehydrogenase or oxidase and said at least one enzyme for at least one substrate is an enzyme with dehydrogenase or oxidase activity.

12. An assay as in claim 11 wherein said products of said at least one enzyme action on said at least one substrate are able to support electrochemiluminescence or chemiluminescence either directly or indirectly.

13. An apparatus for the detection and/or assay of an analyte of interest using electrochemistry, comprising:
an electrical power supply able to deliver a voltage;
a cell able to accept a sample containing structures of conducting and/or semi-conducting material, wherein said cell contains at least two electrodes which are externally and electronically connected to said electrical power supply and said cell is either an integral part of the apparatus or is a removable element,
a detector able to detect electrochemistry said detector selected from the following; film, photomultiplier tube, photodiode, phototransitor, CCD camera, electrochemical cell, wherein said detector does not or is not able to detect electrochemistry from said at least two electrodes.

14. The apparatus of claim 13 wherein the cell comprises at least three functional zones a central zone for the sample to be assayed and at least two zones which flank the central zone which contain said at least two electrodes wherein each electrode of said at least two electrodes is contained within each of said at least two zones which flank the central zone.

15. The apparatus of claim 14 wherein the cell contains a window for the detector such that only the central zone is observable by the detector.

16. The apparatus of claim 14 wherein the central zone of the cell is separated from the flanking zones by at least one membrane which is at least semi-permeable.

17. The apparatus of claim 15 wherein said detector and electrical power supply are controlled by a computer.

18. The apparatus of claim 17 wherein said voltage is in the range of 5V/cm to 30 kV/cm.

19. A kit for carrying out an assay comprising;
a container with a binding species labelled with an electrochemically detectable species
a container with at least one structure of conducting and/or semi-conducting material coupled to a complementary binding species, wherein said at least one structure of conducting and/or semi conducting material is capable of being stimulated to carry our electrochemistry on application of an applied voltage to an electrolyte in contact with said at least one structure of conducting and/or semi conducting material when not in electronic contact with an external electronic control circuit.

20. A kit for carrying out an assay as in claim 19 further containing a container with a bifunctional binding species.

21. A kit for carrying out an assay as in claim 19 where said electrochemically detectable species is able to support electrochemiluminescence or chemiluminescence either directly or indirectly.

22. An immunochromatographic device containing;
a binding species labelled with an electrochemically detectable species,
a binding species immobilised onto a solid phase
and at least one structure of conducting and/or semi-conducting material positioned such that electronic connection to an outside electrical circuit can be avoided.

23. An immunochromatographic device as described in 22 additionally containing at least two electrodes which are able to make electronic contact with an external electronic circuit.

24. A method for activating a stabilised dioxetane to emit light which comprises subjecting said dioxetane to electrochemistry which results in the destabilisation of said stabilised dioxetane causing its chemical decomposition resulting in light emission.

25. A method for activating a stabilised dioxetane to emit light as in claim 24 wherein said electrochemistry is provided by a structure of conducting and/or semi-conducting material not in electronic contact with an external electrical circuit.

26. A method for generating electromagnetic radiation within a separations media which comprises applying a voltage to a separations media which contains or is constructed from structures of conducting and/or semi-conducting material such that said separations media is not rendered conductive and/or semi-conductive.

27. The method as in claim 26 where said separations media is made from the following materials: agarose gels, polyacrylamide gels, cellulose fibres, and derivatives thereof.

28. The method as in claim 26 wherein said electromagnetic radiation is generated by electrochemiluminescence or chemiluminescence.
